# EUROPEAN PATENT SPECIFICATION

(11) **EP 2 209 439 B1**
(45) Date of publication and mention of the grant of the patent: **23.12.2015**
(21) Application number: 08802859.2
(22) Date of filing: 10.10.2008
(51) Int. Cl.: A61B 5/03, A61B 5/11, A61B 5/00, A61B 17/11, A61F 2/04, A61N 1/378

(54) **SYSTEM FOR TREATING A PATIENT HAVING AN INTESTINAL DISORDER**
SYSTEM ZUR BEHANDLUNG EINES PATIENTEN MIT EINER DARMERKRANKUNG
SYSTÈME PERMETTANT DE TRAITER UN PATIENT ATTEINT DE TROUBLES INTESTINAUX

(30) Priority: 11.10.2007 US 960715 P; 11.10.2007 US 960716 P; 12.10.2007 US 960764 P
(43) Date of publication of application: 28.07.2010
(73) Proprietor: Kirk Promotion LTD., 114 79 Stockholm (SE)
(72) Inventor: FORSELL, Peter, CH-6300 Zug (CH)
(74) Representative: Klunker . Schmitt-Nilson . Hirsch
(86) International application number: PCT/EP2008/008588
(87) International publication number: WO 2009/046996

(56) References cited:
- EP-A1- 1 600 183
- WO-A-03/086507
- WO-A-2006/014496
- US-A- 4 728 328
- US-A- 6 050 982
- US-A1- 2004 122 527
- US-A1- 2005 256 587
- US-A1- 2006 195 139
- US-B1- 6 402 767

## Description

### Background of the invention

The present invention relates to a system and method for treating a patient having a disorder related to the patient's intestine. Such disorder may be caused by injury, birth defect, cancer or other diseases, such as constipation or incontinence.

In an attempt to overcome such disorders, many different solutions have been proposed. These solutions often include surgery, in particular where a portion of the intestine has to be removed. The reason for such operation may be colorectal cancer, perforated diverticulitis or other kinds of diseases, such as ulceros colitis or Crohns disease. For instance, in the case of ileostomy, jejunostomy, colostomy and rectostomy operations the small intestine (jejunum or ileum) or the large intestine (colon or rectum) is cut and the open end of the healthy portion of the intestine is reattached either to a surgically created stoma in the patient's abdominal wall or, where possible, to the patient's rectum or anus or to tissue adjacent the patient's anus.

The problem then arises to control the intestinal contents flow and, more particularly, to prevent feces from exiting the patient's body uncontrolled. The patient is typically required to excrete into a colostomy bag. This is obviously inconvenient and, in addition, may cause skin irritation since such a bag arrangement requires an adhesive plate to be attached to the patient's skin in order to render the bag liquid tight.

US patent no. 4,222,377 suggests the use of an inflatable artificial sphincter comprising a cuff around the anal or urethral canal. A manually operated pump is implanted in the patient's scrotum for inflating and deflating the artificial sphincter.

Similarly, US patent no. 5,593,443 discloses an artificial hydraulic anal sphincter under voluntary control. More specifically, the patient may actuate a mechanical or electrical pump for inflating and deflating a cuff. The cuff consists of two parts positioned on opposite sides of the intestine and pressing the intestinal walls together when inflated.

US 6,752,754 B1 discloses an artificial rectum for replacing a portion of a patient's rectum. An inlet of the artificial rectum is operatively connected to the distal end of the patient's large intestine and communicates fecal matter to a macerator-type pump that discharges the feces through an outlet of the artificial rectum connected to the patient's anus. The pump includes a helical screw-type impeller, which when rotated creates shearing effects on the feces, causing it to move down the thread of the screw impeller and discharge through the patient's anus.

US 2004/122527 A1 discloses an implantable prosthetic digestive organ for moving materials through a portion of the digestive tract applied to an artificial large bowel. The implantable prosthetic organ may comprise an implantable pump and a valve. The pump and the valve actuating mechanism may be powered through a coil inductively coupled intracutaneously to an external power source, or by a battery rechargeable through such coil and external power source.

### Summary of the invention

It is an object of the present invention to provide an improved system for treating a patient having a disorder related to the patient's intestine.

### ARTIFICIAL INTESTINE SECTION WITH WIRELESSLY CHARGED ACCUMULATOR AND BULGE STRUCTURE FOR FRONTAL ATTACHMENT TO INTESTINE

A system according to the invention for treating a patient having a disorder related to the patient's intestine comprises an artificial intestine section adapted to being implanted inside a patient's body along with an accumulator for accumulating energy. The intestine section of the present invention has a first open end portion and a second open end portion in flow communication with one another, wherein at least the first open end portion and possibly also the second open end portion is adapted to being connected to a surgically created opening in the patient's intestine. Furthermore, the accumulator is adapted to be charged wirelessly with energy and to be arranged so as to supply energy directly or indirectly to at least one energy consuming part of said artificial intestine section. At least the first open end portion is adapted to be connected to a cross-sectional opening surgically created in the patient's intestine. The open end portion of the artificial intestine section comprises
- a conduit having an outer surface with at least one bulge extending outwardly from the conduit's outer surface in a circumferential direction of the conduit about at least a part of the conduit's circumference, and
- a blocking ring loosely fitting over the outer surface of the conduit with a clearance between the outer surface and the blocking ring for mounting intestinal tissue within the clearance, said blocking ring having an inner cross sectional
diameter which is smaller than or substantially identical to an outer cross sectional diameter of the at least one bulge so as to prevent the blocking ring from slipping over the bulge when intestinal tissue is mounted within the clearance. The artificial intestine section may then be affixed to the cross-sectional opening of the intestine part by inserting the artificial intestine section having a bulge formed on the outside thereof into the cross-sectional opening of the intestine part so that the intestine part extends over the bulge from one side of the bulge and advancing a blocking ring over the intestine part towards the bulge from the respective other side of the bulge such that the intestine part is located intermediate the outer surface of the artificial intestine section and the blocking ring.

### LATERAL CONNECTION TO INTESTINE

Preferably, the second open end portion of the artificial intestine section is adapted to being connected to a lateral opening surgically created in a wall of the patient's intestine. Thus, rather than connecting the artificial intestine section to the cross-sectional end of the patient's intestine, it is connected to a lateral opening in the patient's intestinal wall, and for this purpose the respective open end portion of the artificial intestine section is specifically adapted.

By connecting the artificial intestine section laterally to the intestine, forces caused by the peristaltic movement of the intestine and acting on the artificial intestine section of the intestine are largely avoided. More specifically, where the artificial intestine section is connected to the cross-sectional opening of the intestine, the peristaltic waves of the intestine tend to pull the intestine off of the connection between the intestine and the artificial intestine section. As compared to this, where the artificial intestine section is attached to an opening in the lateral wall of the intestine, the peristaltic waves pass the artificial intestine section substantially without any impact on the connection between the intestinal wall and the artificial intestine section.

### STRUCTURE OF LATERAL ATTACHMENT

In order to securely attach the artificial intestine section to the lateral opening, the second open end portion may comprise a shoulder portion formed around the end portion for lateral connection to the patient's intestinal wall. Preferably, at least a part of the shoulder portion extends laterally from the artificial intestine section by 3 mm to 20 mm. Furthermore, the shoulder portion preferably has a curved cross section, so as to generally conform to the intestinal wall when laterally attached thereto. An open end portion adapted in this way can advantageously be attached to the intestinal wall from the outside thereof.

According to an improved embodiment, the shoulder portion may be split into an upper and a lower shoulder portion with a gap between the upper and lower shoulder portions for accommodating intestinal wall tissue therein. The lower shoulder portion, if suitably adapted, can then be placed inside the patient's intestine through the surgically created lateral wall opening, whereas the upper shoulder portion will be placed outside the intestinal wall.

In order to allow the lateral wall opening to be easily stretched over the lower shoulder portion when the lower shoulder portion is advanced there through and yet in order to have a large contact area between the intestinal wall and the shoulder portion, the upper shoulder portion may be made larger than the lower shoulder portion. Thus, the surface area of the upper shoulder portion contacting the intestinal wall is also larger than the surface area of the lower shoulder portion contacting the intestinal wall.

The open end portion for lateral connection to the patient's intestinal wall may be adapted to being connected to the patient's intestinal wall by gluing. For instance, it may have a particular rough surface structure for the glue to better adhere. Also, the open end portion may be adapted to being connected to the patient's intestinal wall by sewing. For instance, a certain area of the shoulder portion may be perforated for stitching through the perforations or may be made from a material which is easy to penetrate with a needles. Similarly, the open end portion may specifically be adapted to being connected to the patient's intestinal wall by stapling.

### SLEEVE STRUCTURE FOR FRONTAL ATTACHMENT TO INTESTINE

The second open end portion may comprise a conduit having an outer surface and a flexible sleeve adapted to axially extend and closely fit around at least part of said outer surface of the conduit. The flexible sleeve can be mounted on said outer surface either folded or rolled upon itself or so as to be foldable upon itself. In either case, the open end portion of the artificial intestine section may then be affixed to the cross-sectional opening of the intestine part by inserting the open end portion of the artificial intestine section into the cross-sectional opening of the intestine part and placing the flexible sleeve so as to extend over both the intestine part and open end portion of the artificial intestine section such that the intestine part is located intermediate the sleeve and the outer surface of the artificial intestine section.

Where the flexible sleeve is mounted on the outer surface of the open end portion of the artificial intestine piece so as to be foldable upon itself, the step of placing the flexible sleeve so as to extend over both the intestine part and open end portion of the artificial intestine section comprises folding the flexible sleeve upon itself such that the intestine part is located intermediate the folded sleeve.

The bulge and sleeve may also be provided on one conduit so as to be used in combination.

The afore-mentioned conduit of the artificial intestine section with a sleeve or with a bulge serve to improve the strength of the connection against axial forces which may e.g. result from the peristaltic movement of the intestine and tend to pull on the intestine.

Furthermore, the conduit of the open end portion preferably comprises a multilayer material. For instance, it is advantageous when the open end portion comprises a porous ingrowth layer that allows ingrowth of living tissue. The ingrowth layer may have a net-like structure and is most preferably made from Dacron®.

### THROUGH-FLOW ARRANGEMENT

### STRAIGHT CONNECTION

According to the afore-described structures of the first and second open end portions of the artificial intestine section, it is possible to connect both the first and second open end portions to a surgically created cross-sectional opening in the patient's intestine, so as to form an intermediate intestine section, or to connect the second open end portions to a surgically created lateral opening in a wall of the patient's intestine.

### STOMA/ANUS CONNECTION

Alternatively, the second open end portion may be adapted to being connected to an surgically created stoma or to the patient's rectum or anus or to tissue adjacent the patient's anus, so as to form an intestine end section.

### DOWNSTREAM CONNECTION TO RESIDUAL INTESTINE

Further alternatively, the second open end portion may be adapted to being connected to a portion of the patient's small intestine or to a portion of the patient's large intestine, as the case may be, and this portion of the patient's intestine may then lead to the surgically created stoma or to the patient's rectum or anus or to tissue adjacent the patient's anus.

### MATERIAL

Preferably, at least the first open end portion is made from a biocompatible material. The biocompatible material of the open end portion may comprise at least one material of the following group of materials: titanium, stainless steel, ceramics, biocompatible polymer material. More specifically, the biocompatible polymer material may comprise at least one polymer of the following group of polymers: polytetrafluoroethylene, silicone, polyurethane, expanded polytetrafluoroethylene (ePTFE).

### INTEST. CONTENT INTERACTING DEVICE AS AN ENERGY CONSUMING PART

In a preferred embodiment, the at least one energy consuming part of the artificial intestine section comprises at least one element adapted to directly or indirectly interact with intestinal contents contained in the artificial intestine section between the first and second open end portions thereof.

### FLOW CONTROL DEVICE AS AN ENERGY CONSUMING PART

In a more advanced embodiment, the at least one element may comprise a flow control device adapted to control flow of intestinal contents from the artificial intestine section through the second open end portion. The flow control device is preferably adapted to prevent flow of intestinal contents from the artificial intestine section through the second open end portion.

### EXIT VALVE AS FLOW CONTROL DEVICE

The flow control device preferably comprises at least one valve, including an exit valve preventing intestinal contents flow through the second open end portion in its closed position. Preferably, the exit valve is a normally closed valve so that no energy is needed to keep the valve closed during the system's inactive periods.

### ENTRY VALVE AS AN ADDITITONAL PART OF THE FLOW CONTROL DEVICE

In addition, the flow control device may comprise an entry valve allowing intestinal contents to flow towards the reservoir in its open position. This can be advantageous particularly during the emptying of the reservoir, when the entry valve should be closed. Therefore, the entry valve is preferably a normally open valve. Accordingly, the exit valve and the entry valve are preferably adapted to cooperate such that when one of the two valves is closed, the respective other valve is open, and vice versa.

### VALVE TYPES

As regards the various valve types that may be employed, the at least one valve may e.g. comprise a compartment with a variable volume adapted to open and close the valve by changing the compartment's volume. Advantageously, the at least one valve comprises at least one passage for filling and emptying the compartment with hydraulic fluid. The compartment preferably has at least one flexible wall defining an opening for a conduit to pass through, the opening being adapted to close upon increase of the compartment's volume.

According to a different embodiment, the at least one valve may be a flap valve. The flap valve may for instance comprise a rotatable disc.

### EXTRA VALVE SEPARATE FROM ARTIFICIAL INTESTINE PIECE

While the valve or valves preferably make an integral part of the artificial intestine section, the artificial intestine section may further comprise one or more extra valves adapted to control flow of intestinal contents in a natural section of the patient's intestine upstream and/or downstream the artificial intestine section. The extra valve may be rigidly connected to the artificial section but may as well form a completely separate part. The extra valve is adapted to being implanted inside the patient's body outside a section of the patient's natural intestine and comprises at least one element adapted to act on the natural intestine section from the outside thereof so as to prevent intestinal contents flow through the natural intestine section. This valve arrangement does not require any surgery on the respective part of the natural intestine when the valve is implanted.

The extra valve may comprise at least one electrical stimulation device adapted to electrically stimulate muscle or neural tissue of an intestine section so as to cause at least partial contraction of the natural intestine section. This is a very gender way of constricting the intestine. The stimulation device preferably comprises at least one electrode adapted to apply electric pulses to the natural intestine section.

It is particularly advantageous to make use of a stimulation device which is adapted to stimulate different portions of the intestine section over time. Thus, different portions of the intestine section can be constricted by stimulation at different times in any predetermined stimulation pattern, thereby giving the intestine portions currently not stimulated time to recover and, thus, improving the blood circulation in the respective intestine section.

Furthermore, the stimulation device can specifically be adapted to stimulate, over time, the different portions of the intestine section in a wave like manner in a direction opposite to natural intestinal contents flow. As a result, the valve counteracts the natural intestinal contents flow, thereby improving the valve's closing function.

Alternatively, or preferably in addition to the stimulation device, the at least one valve may comprise a constriction device implanted in the patient's body for at least partly constricting the natural intestine section mechanically from outside the natural intestine section. Where the stimulation device is combined with the constriction device, the stimulation device and the constriction device preferably act on the same intestine section. In that case, it is advantageous if the constriction device in its normal condition constricts the natural intestine section only partly, in order not to damage the intestine over time. Complete constriction and, thus, closing of the intestine may then be obtained by additionally stimulating the natural intestine section in a manner as described before.

In addition, when constriction of the intestine section caused by the constriction device is released, the stimulation device may, if accordingly adapted, be used to pump intestinal contents along the natural intestine section by, over time, stimulating the different portions of the natural intestine section in a wave like manner in a direction of natural intestinal contents flow. In this situation, the valve may incorporate the additional function of a pump for actively supporting the discharge of feces from the human body.

### PUMP AS AN ENERGY CONSUMING PART

The at least one energy consuming part of the artificial intestine section may comprise a pump for advancing intestinal contents through the second open end portion to outside the artificial intestine section.

### PUMP WITH RESERVOIR

In addition to the pump, the artificial intestine section may comprise a reservoir between the first and second open end portions for receiving and temporarily collecting therein intestinal contents supplied through the first open end portion. In this case, the pump is preferably adapted for emptying the reservoir through the second open end portion. A variety of different structures may be realized.

For instance, the reservoir may be formed by a bellow, said bellow having an end wall closing the bellow at one end thereof. The end wall may then make part of the pump such that a volume of the bellow is reduced upon advancement of said end wall. Preferably, bellow is made of a resilient material so as to urge the bellow into a normally expanded position.

In another embodiment, the pump may comprise a movable piston, with a front end of the piston extending into the reservoir such that a volume of the reservoir is reduced upon advancement of the piston. Preferably, the piston is spring loaded so as to urge the piston into a normally retracted position.

Alternatively, the pump may be adapted for being permanently arranged inside the reservoir.

In a further alternative, the reservoir may have a flexible wall and the pump is adapted for emptying the reservoir by squeezing the reservoir. In this case, the pump may e.g. include a constriction device adapted to alternately constrict and release sections of the reservoir so as to pump intestinal contents along the reservoir by, over time, constricting different sections of the reservoir in a wave like manner. More specifically, the reservoir may have a tube-like form and a roller pump may be used as the pump acting on the tube-like reservoir from the outside thereof.

### MOTOR AS AN ENERGY CONSUMING PART

Where the valves or pump or any other energy consuming part of the artificial intestine section is not or not only manually drivable, the artificial intestine section may comprise at least one motor arranged for automatically driving at least one energy consuming part of the artificial intestine section. The motor is preferably arranged to be driven by electric or electromagnetic energy.

A motor in the sense of the present invention is a device that transforms energy other than mechanical energy into mechanical energy. While a pump in the sense of the present invention is a device for advancing liquid or pasty material, a pump may at the same time be a motor in certain circumstances, such as where the transformation of energy into mechanical energy causes advancement of the liquid or pasty material without any intervening mechanical means such as a piston, bellow or the like.

For instance, the at least one motor can be arranged for driving at least one of the valve or valves, respectively, between its closed and open position. Also, the at least one motor can be arranged for driving the pump.

A manually operable switch may be provided for activating the at least one motor, the switch being preferably arranged for subcutaneous implantation so as to be operable from outside the patient's body.

### ENERGY TRANSMISSION

Where energy is not transmitted wirelessly, galvanic coupling elements may be provided at least between the accumulator and the energy consuming part, in particular the motor, for transmitting energy to the motor in contacting fashion.

The energy may alternatively be transmitted wirelessly from the accumulator to the motor. Thus, the energy source may comprise a wireless energy transmitter adapted to wirelessly transmit energy from the accumulator to the energy consuming part.

Preferably, in order to reduce the number of parts and possibly increase the system's efficiency, the energy consuming part , in particular the motor, can be adapted to directly transform the wirelessly transmitted energy from the accumulator into kinetic energy. In the alternative, the energy consuming part will have to comprise a transforming device for transforming the wirelessly transmitted energy from the accumulator into electric energy.

Similarly, the system preferably comprises an implantable energy transforming device for transforming the wirelessly transmitted energy from outside the patient's body into energy to be stored in the accumulator of the implanted system and further comprises a wireless energy transmitter adapted to wirelessly transmit energy from outside the patient's body to said implantable energy transforming device.

It is further preferred to set up the system such that the energy consuming part is driven with the electric energy, as said energy transforming device transforms the wireless energy into the electric energy.

The energy transmitter can be adapted to generate an electromagnetic field, a magnetic field or an electrical field. The wireless energy may be transmitted by the energy transmission device by at least one wireless signal. More specifically, the energy transmitter may be adapted to transmit the energy by at least one wireless energy signal, which may comprise an electromagnetic wave signal, including at least one of an infrared light signal, a visible light signal, an ultra violet light signal, a laser signal, a microwave signal, an X-ray radiation signal, and a gamma radiation signal. Also, the wireless energy signal may comprise a sound or ultrasound wave signal. Furthermore, the wireless energy signal may comprise a digital or analog signal or a combination thereof.

### ENERGY TRANSMISSION FEEDBACK

A feedback subsystem, which can make part of a control device described subsequently, can advantageously be provided to wirelessly send feedback information related to the energy to be stored in the accumulator from inside the human body to the outside thereof. The feedback information is then used for adjusting the amount of wireless energy transmitted by the energy transmitter. Such feedback information may relate to an energy balance which is defined as the balance between an amount of wireless energy received inside the human body and an amount of energy consumed by the at least one energy consuming part. Alternatively, the feedback information may relate to an energy balance which is defined as the balance between a rate of wireless energy received inside the human body and a rate of energy consumed by the at least one energy consuming part.

### ACCUMULATOR

The accumulator preferably comprises a rechargeable battery. It may alternatively or in addition comprise a capacitor. The accumulator may be adapted for being implanted inside the patient's body either fixedly connected to the artificial intestine section or distant to the artificial intestine section.

### PRIMARY ENERGY SOURCE

A primary energy source may be provided for charging the accumulator with energy from outside the patient's body. The primary energy source is preferably adapted to being mounted on the patient's body.

### CONTROL UNIT

It is advantageous to provide a control unit adapted to directly or indirectly control one or more elements of the system, such as for controlling opening of the exit valve and/or closing of the entry valve, in particular in a manner such that when one of the two valves is closed, the respective other valve is open, and vice versa. The control unit can also be adapted to control actuation of the pump.

The control unit is preferably operable by the patient, e.g. particularly in order to empty the reservoir.

At least part of the control unit may be adapted to be implantable in the patient's body. For instance, a manually operable switch may be provided for activating the control unit, the switch preferably being arranged for subcutaneous implantation so as to be operable from outside the patient's body. Also, the control unit may comprise a first part adapted for implantation in the patient's body and a second part adapted to cooperate with the first part from outside the patient's body. In this case, the control unit can be adapted to transmit data from the external second part of the control unit to the implanted first part of the control unit in the same manner as energy is transmitted by said wireless energy transmitter from outside the patient's body to said implantable energy transforming device.

That is, the second part of the control unit may be adapted to wirelessly transmit a control signal to the implantable first part of the control unit for controlling the at least one energy consuming part from outside the patient's body. Also, the implantable first part of the control unit may be programmable via the second part of the control unit. Furthermore, the implantable first part of the control unit may be adapted to transmit a feedback signal to the second part of the control unit.

### SENSOR

Furthermore, a physical parameter sensor adapted to directly or indirectly sense a physical parameter of the patient can be provided. The physical parameter sensor may be adapted to sense at least one of the following physical parameters of the patient: a pressure within the artificial intestine section, a pressure within the patient's natural intestine, an expansion of the artificial intestine section, a distension of an intestinal wall of the patient's natural intestine, a movement of the patient's intestinal wall.

Similarly, a functional parameter sensor adapted to directly or indirectly sense a functional parameter of the system can be provided, wherein the functional parameter sensor may be adapted to sense at least one of the following functional parameters of the system: a pressure against a part of the system such as the artificial intestine section, a distension of a part of the system such as a wall of the artificial intestine section, an electrical parameter such as voltage, current or energy balance, a position or movement of a movable part of the system.

Preferably, an indicator is coupled to the sensor or sensors, the indicator being adapted to provide a signal when a sensor senses a value for the parameter beyond a predetermined threshold value. The sensor signal may comprise at least one of the following types of signals: a sound signal, a visual signal.

### METHOD OF TREATMENT (IMPLANTATION)

The invention does not only relate to the system described above, but also to a method of treating a patient having a disorder related to the patient's intestine, more specifically to a method of implanting the system and to a method of using the system once it is installed on the patient.

Accordingly, a surgical method of treating a patient according to the invention comprises the steps of:
- cutting the patient's skin and abdominal wall,
- dissecting an area of the patient's intestine,
- surgically creating at least one opening in the dissected intestinal area so as to create an artificial intestinal opening,
- providing an artificial intestine section having a first open end portion and a second open end portion in flow communication with one another and affixing the first open end portion to the artificial intestinal opening so as to be in flow communication therewith,
- providing an accumulator adapted to be charged wirelessly with energy from outside the patient's body and arranging the accumulator inside the patient's body so as to allow for supplying energy directly or indirectly from the accumulator to at least one energy consuming part of said artificial intestine section, and
- suturing the abdominal wall and skin.

A corresponding laparoscopic surgical method of treating a patient comprises the steps of:
- making a small opening in the patient's skin and abdominal wall,
- introducing a needle in the abdominal cavity,
- inflating the abdominal cavity with gas,
- inserting at least one trocar into the cavity,
- introducing a camera through the trocar,
- inserting at least one dissecting instrument preferably through a second trocar,
- dissecting an area of the intestine,
- surgically creating at least one opening in the dissected intestinal area so as to create an artificial intestinal opening,
- providing an artificial intestine section having a first open end portion and a second open end portion in flow communication with one another and affixing the first open end portion to the artificial intestinal opening so as to be in flow communication therewith,
- providing an accumulator adapted to be charged wirelessly with energy from outside the patient's body and arranging the accumulator inside the patient's body so as to allow for supplying energy directly or indirectly from the accumulator to at least one energy consuming part of said artificial intestine section, and
- extracting the instruments, camera and trocar, and in relation thereto
- suturing, if necessary, the abdominal wall and permanently closing the skin.

Where the accumulator is provided fixedly connected to the artificial intestine section, it may be arranged inside the patient's body along with the artificial intestine section. Alternatively, where the accumulator is provided separate from the artificial intestine section, it may be implanted inside the patient's body distant to the artificial intestine section.

Where energy is not transferred wirelessly from the accumulator to the energy consuming part of the artificial intestine section, the accumulator may be galvanically coupled to the artificial intestine section for transmitting the energy to the at least one energy consuming part of the artificial intestine section in contacting fashion.

### DIVIDING THE INTESTINE

The method of implantation may involve dividing the intestine so as to obtain one or two cross-sectional opening of the intestine. The method of implantation may then comprise the following additional steps:
- dissecting a portion of the dissected intestinal area downstream of the intestinal opening to be created, such that intestinal mesentery connected to the dissected portion is opened in such a way that supply of blood through the mesentery to the dissected intestinal area is maintained as far as possible on both sides of the dissected portion, and
- dividing the patient's intestine in the dissected portion so as to create an upstream part of the intestine with an artificial intestinal opening being formed as a cross-sectional opening at the downstream side thereof and a downstream part of the intestine with an artificial intestinal opening being formed as a cross-sectional opening at the upstream side thereof, wherein the mesentery maintains a tissue connection between the upstream and downstream intestine parts.

### FRONTAL CONNECTION AT UPSTREAM END

At least the first open end portion of the artificial intestine section can then be affixed to the cross-sectional downstream opening of the upstream intestine part so as to be in flow communication therewith.

### FRONTAL CONNECTION AT DOWNSTREAM END

Also, the second open end portion of the artificial intestine section can be affixed to the cross-sectional upstream opening of the downstream intestine part so as to be in flow communication therewith.

### FRONTAL CONNECTION AT BOTH ENDS

The step of affixing the open end portion of the artificial intestine section to the cross-sectional opening of the intestine part comprises:
- inserting the artificial intestine section having a bulge formed on the outside thereof into the cross-sectional opening of the intestine part so that the intestine part extends over the bulge from one side of the bulge, and
- advancing a blocking ring over the intestine part towards the bulge from the respective other side of the bulge such that the intestine part is located intermediate the outer surface of the artificial intestine section and the blocking ring.

The afore-mentioned open end portion of the artificial intestine section with a sleeve or with a bulge serve to improve the strength of the connection against axial forces which may e.g. result from the peristaltic movement of the intestine and tend to pull on the intestine. The open end portion of the artificial intestine section may also combine a sleeve and a bulge.

### LATERAL CONNECTION UPSTREAM, FRONTAL CONNECTION DOWNSTREAM

It has been described above that the second open end portion of the artificial intestine section may be connected to a lateral opening in the wall of the downstream intestine part while the first open end portion is connected to a cross-sectional opening of the upstream intestine part. The connection can alternatively be made vice versa, in which case the method of implantation may comprise the following steps:
- closing the cross-sectional opening at the upstream side of the downstream intestine part,
- surgically creating an opening in a wall of the downstream intestine part so as to create a lateral intestinal opening therein,
- affixing the second open end portion of the artificial intestine section to the lateral intestinal opening of the downstream intestine part so as to be in flow communication therewith and
- affixing the first open end portion of the artificial intestine section to the cross-sectional downstream opening of the upstream intestine part so as to be in flow communication therewith.

Alternatively, or in addition, the step of affixing the open end portion of the artificial intestine section to the cross-sectional opening of the intestine part comprises:
- inserting the artificial intestine section having a bulge formed on the outside thereof into the cross-sectional opening of the intestine part so that the intestine part extends over the bulge from one side of the bulge, and
- advancing a blocking ring over the intestine part towards the bulge from the respective other side of the bulge such that the intestine part is located intermediate the outer surface of the artificial intestine section and the blocking ring.

The afore-mentioned open end portion of the artificial intestine section with a sleeve or with a bulge serve to improve the strength of the connection against axial forces which may e.g. result from the peristaltic movement of the intestine and tend to pull on the intestine. The open end portion of the artificial intestine section may also combine a sleeve and a bulge.

### LATERAL CONNECTION UPSTREAM, FRONTAL CONNECTION DOWNSTREAM

It has been described above that the second open end portion of the artificial intestine section may be connected to a lateral opening in the wall of the downstream intestine part while the first open end portion is connected to a cross-sectional opening of the upstream intestine part. The connection can alternatively be made vice versa, in which case the method of implantation may comprise the following steps:
- closing the cross-sectional opening at the upstream side of the downstream intestine part,
- surgically creating an opening in a wall of the downstream intestine part so as to create a lateral intestinal opening therein,
- affixing the second open end portion of the artificial intestine section to the lateral intestinal opening of the downstream intestine part so as to be in flow communication therewith and
- affixing the first open end portion of the artificial intestine section to the cross-sectional downstream opening of the upstream intestine part so as to be in flow communication therewith.

### LATERAL CONNECTION AT BOTH ENDS

Of course, it is also possible and even preferred to connect both open end portions of the artificial intestine piece to lateral openings in the intestinal wall, in which case the method of implantation may comprise the following steps:
- closing the cross-sectional opening at the downstream side of the upstream intestine part,

### STRUCTURE OF LATERAL ATTACHMENT

Where the open end portion of the artificial intestine section is to be affixed to a lateral intestinal opening so as to be in flow communication therewith, this may comprise the step of connecting the afore-mentioned shoulder portion, which is formed around the open end portion, to the patient's intestinal wall so as to surround the lateral intestinal opening. In particular, the step of affixing the open end portion to the intestine may comprise attaching the shoulder portion to the patient's outer intestinal wall.

Alternatively, where the shoulder portion is split into an upper and a lower shoulder portion with a gap between the upper and lower shoulder portions, the step of affixing the open end portion of the artificial intestine section to the lateral intestinal opening comprises placing the lower shoulder portion inside the patient's intestine and the upper shoulder portion outside the patient's intestine such that intestinal wall tissue is accommodated in the gap.

The step of affixing the open end portion of the artificial intestine section to the lateral intestinal opening may comprise gluing, sewing and/or stapling the open end portion to the patient's intestinal wall.

### EXIT THROUGH STOMA

As mentioned before, the downstream intestinal part may be connected to a surgically created stomy or to the patient's rectum or anus or to tissue adjacent the patient's anus. In the case of a connection to a stomy, this would involve the following steps:
- cutting the patient's skin and abdominal wall so as to create an opening for an intestinal stomy,
- dissecting the area of the stomy opening,
- dividing the intestine at a location downstream of the artificial intestine section so as to create an upstream natural intestine section having a cross-sectional opening at the downstream end thereof and a downstream natural intestine section,
- dissecting the mesentery of the upstream natural intestine section in the area of the cross-sectional opening thereof to prepare for creating the intestinal stomy,
- advancing the downstream end of the upstream natural intestine section through the abdominal wall and skin, and
- suturing the cross-sectional opening of the upstream natural intestine section to the skin with the intestinal mucosa turned inside out, thereby achieving the intestinal stomy.

### EXIT THROUGH ANUS

In the case of a connection to the patient's anus or to tissue adjacent the patient's anus, this would involve the following steps:
- dividing the intestine at a location downstream of the artificial intestine section so as to create an upstream natural intestine section having a cross-sectional opening at the downstream end thereof and a downstream natural intestine section leading to the patient's anus,
- dissecting the area of the patient's anus and surgically separating the downstream natural intestine section from the patient's anus, whereas the steps of dividing the intestine and separating the intestine section leading to the patient's anus can alternatively be carried out in reversed order,
- dissecting the mesentery of the upstream natural intestine section in the area of the cross-sectional opening at the downstream end thereof to prepare for connecting the upstream natural intestine section to the patient's anus or tissue adjacent the patient's anus,
- advancing the downstream end of the upstream natural intestine section through the patient's anus, and
- suturing the cross-sectional opening of the upstream natural intestine section to the patient's anus or tissue adjacent the patient's anus.

Depending upon the circumstances, the step of dividing the intestine so as to form the upstream natural intestine section may be performed either on the patient's small intestine or on the patient's large intestine.

### INTEST. CONTENT INTERACTING DEVICE AS AN ENERGY CONSUMING PART (FLOW CONTROL DEVICE / EXIT VALVE / ENTRY VALVE / PUMP / RESERVOIR)

As mentioned before, the artificial intestine section or system may comprise at least one energy consuming part adapted to directly or indirectly interact with intestinal contents contained in the artificial intestine section between the first and second open end portions thereof. This element will be implanted along with the artificial intestine section. As also mentioned before, the energy consuming part may comprise a flow control device adapted to control flow of intestinal contents from the artificial intestine section through the second open end portion.

Again, the flow control device may comprise an exit valve preventing intestinal contents flow through the second open end portion in its closed position and may additionally comprise an entry valve allowing intestinal contents to flow through the first open end portion into the artificial intestine section in its open position.

Alternatively or in addition, as also mentioned before, the flow control device may comprise a pump for advancing intestinal contents through the second open end portion to outside the artificial intestine section.

If a reservoir is provided between the first and second open end portions for receiving and temporarily collecting therein intestinal contents supplied through the first open end portion, the pump may be adapted to empty the reservoir through the second open end portion.

Furthermore, where the pump comprises a manually operable switch for activating the pump, the method of implantation may further comprise the step of implanting the switch subcutaneously so as to be operable from outside the patient's body.

### MOTOR

Again, at least one motor may be implanted along with the artificial intestine section and may be arranged for automatically driving one or more energy consuming part of the flow control device. Where the motor comprises a manually operable switch for activating the motor, the method of implantation may further comprise the step of implanting the switch subcutaneously so as to be operable from outside the patient's body.

### ENERGY TRANSMISSION

Where energy is transmitted wirelessly, for instance from outside the patient's body to inside the patient's body either to an energy consuming part and/or more specifically to be stored in the accumulator or from the accumulator to the energy consuming part, it may further be necessary to implant an energy transforming device for transforming the wireless energy into electric energy. Alternatively or in addition, galvanic coupling elements may be implanted, e.g. for transmitting energy to the energy consuming part in contacting fashion from the implanted energy source.

### ACCUMULATOR

Preferably, at least one rechargeable battery is provided as the accumulator. Alternatively or in addition thereto, at least one capacitor may be provided as the accumulator.

### CONTROL UNIT

Furthermore, as mentioned previously, at least a part of a control unit may be implanted inside the patient's body adapted to directly or indirectly control one or more of the elements that have also been implanted in the patient's body. Where the control unit comprises a manually operable switch for activating the control unit, the method of implantation may further comprise the step of implanting said switch subcutaneously so as to be operable from outside the patient's body.

### SENSOR

As mentioned before, one or more physical and/or functional parameter sensors may be implanted to directly or indirectly sense physical and/or functional parameters inside the patient and in the system implanted inside the patient. Where the sensor is a pressure sensor, it may be placed in the artificial intestine section or the patient's natural intestine so as to sense the pressure within the artificial intestine section or patient's natural intestine, respectively. Where the sensor is a tension sensor, it may be placed in contact with the artificial intestine section or the patient's natural intestine so as to sense an expansion of the artificial intestine section or patient's natural intestine, respectively. Where the sensor is a movement sensor, it may be placed in contact with the artificial intestine section or the patient's natural intestine so as to sense movement of the artificial intestine section or patient's natural intestine, respectively. The functional sensor may be adapted to measure at least one of the following functional parameters: an electrical parameter such as voltage, current or energy balance or a stimulation parameter in relation to the system.

### USE

Once the system according to the invention has been properly installed and where the at least one energy consuming part of the artificial intestine section comprises at least one element adapted to directly or indirectly interact with intestinal contents contained in the artificial intestine section between the first and second open end portions thereof, the at least one element may be activated so as to interact with the intestinal contents.

### EXIT AND ENTRY VALVE

Where the at least one element comprises an exit valve preventing intestinal contents flow from the artificial intestine section through the second open end portion in its closed position, the method of use may further comprise the steps of opening the exit valve and then removing intestinal contents from the artificial intestine section. Furthermore, where the at least one element further comprises an entry valve allowing intestinal contents to flow through the first open end portion into the artificial intestine section in its open position, the method may further comprise the step of closing the entry valve before removing intestinal contents from the artificial intestine section.

### PUMP

Where the at least one element interacting with intestinal contents inside the artificial intestine piece comprises a pump, the method of use may further comprise the step of advancing intestinal contents from the artificial intestine section through the second open end portion thereof to outside the artificial intestine section by means of the pump. The pump may be activated by manually operating a subcutaneously arranged actuator from outside the patient's body.

### PUMP WITH RESERVOIR

More specifically, if the artificial intestine section comprises a reservoir between the first and second open end portions for receiving and temporarily collecting therein intestinal contents supplied through the first open end portion, the step of advancing intestinal contents to outside the intestine section may comprise operating the pump so as to empty the reservoir.

Where the reservoir is formed by a bellow, said bellow having an end wall closing the bellow at one end thereof and said end wall making part of the pump, the step of advancing the intestinal contents to outside the intestine section may comprise advancing the end wall so as to reduce a volume of the bellow.

Where the pump comprises a movable piston with a front end of the piston extending into the reservoir, the step of advancing the intestinal contents to outside the intestine section may comprise advancing the piston so as to reduce a volume of the reservoir.

Where the reservoir has a flexible wall, the step of advancing the intestinal contents to outside the intestine section may comprise squeezing the reservoir by means of the pump. For instance, the pump may include a constriction device, in which case the step of advancing the intestinal contents to outside the intestine section may comprise alternately constricting and releasing sections of the reservoir so as to pump the intestinal contents along the reservoir by, over time, constricting different sections of the reservoir in a wave like manner. More specifically, the reservoir may have a tube-like form and the pump can be a roller pump acting on the tube-like reservoir from the outside thereof.

### MOTOR

Furthermore, where the at least one element interacting with intestinal contents inside the artificial intestine piece comprises a motor, the method of use may further comprise the step of driving at least the exit valve between its closed and open positions and/or driving at least the pump by means of the motor. In either case, the motor is preferably activated by manually operating a subcutaneously arranged actuator from outside the patient's body.

### ENERGY

Again, energy is to be transmitted wirelessly from outside the patient's body to the accumulator. Therefore, upon charging the accumulator with energy, the method of use involves the following steps:
- transmitting the energy wirelessly from outside the patient's body,
- transforming the wirelessly transmitted energy into energy to be stored in the accumulator by means of an energy transforming device implanted in the patient's body, and
- charging the accumulator with at least part of the transformed energy.

The method of use may then further involve the step of supplying energy from the accumulator to at least one implanted energy consuming part of the system, preferably wirelessly.

More preferably, at least part of the wirelessly transmitted energy is transformed into electric energy and used for the energy consuming part of the system, as said part of the wirelessly transmitted energy is transformed into the electric energy.

As mentioned before, the wireless energy can advantageously be transmitted by means of an electromagnetic field, a magnetic field or an electrical field. More specifically, the energy may be transmitted by at least one wireless energy signal, which may comprise an electromagnetic wave signal, including at least one of an infrared light signal, a visible light signal, an ultra violet light signal, a laser signal, a microwave signal, an X-ray radiation signal, and a gamma radiation signal. Also, the wireless energy signal may comprise a sound or ultrasound wave signal. Furthermore, the wireless energy signal may comprise a digital or analog signal or a combination thereof.

### CONTROL

Where a first part of a control unit for controlling at least one energy consuming part of the system is implanted inside the patient's body, the method of use may further comprise the step of using the external second part of the control unit to transmit data to the implanted first part of the control unit. Preferably, the data are transmitted to the implanted first part of the control unit in the same manner as energy is transmitted to the implanted energy consuming part. More particularly, the data are preferably transmitted wirelessly to the implanted first part of the control unit. This may involve a wireless control signal.

For instance, the implanted first part of the control unit can be programmed via the external second part of the control unit. Furthermore, a feedback signal may be transmitted from the implanted first part of the control unit to the external second part of the control unit.

### SENSOR

Where one or more of the afore-mentioned sensors are provided, the method of use may comprise the step of sensing a physical parameter in the patient's body and/or a functional parameter of the artificial intestine piece or system in the patient's body, such as one or more of the following parameters: a pressure within the artificial intestine section, a pressure within the patient's natural intestine, an expansion of the artificial intestine section, a distension of an intestinal wall of the patient's natural intestine, a movement of the patient's intestinal wall, a pressure against a part of the system such as the artificial intestine section, a distension of a part of the system such as a wall of the artificial intestine section, an electrical parameter such as voltage, current or energy balance, a position or movement of a movable part of the system.

A signal, such as a sound signal or a visual signal, may be provided when a value for the physical parameter sensed is beyond a predetermined threshold value.

The invention will now be described in more detail in context with some preferred embodiments of the invention as shown in the accompanying drawings.

### Brief description of the drawings

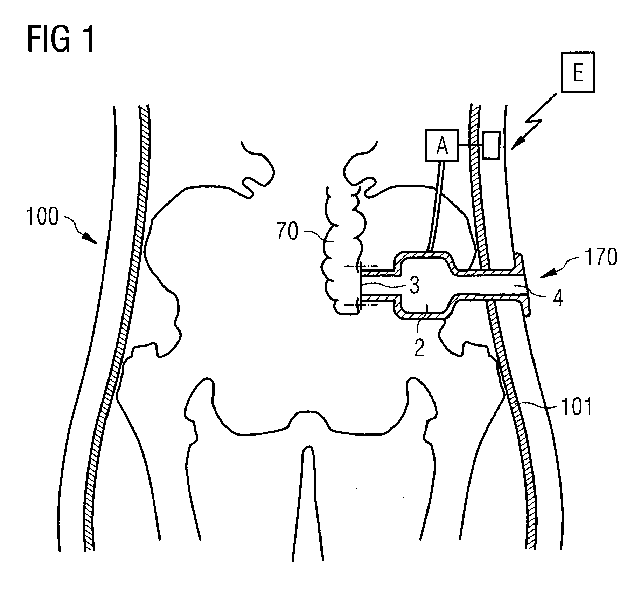

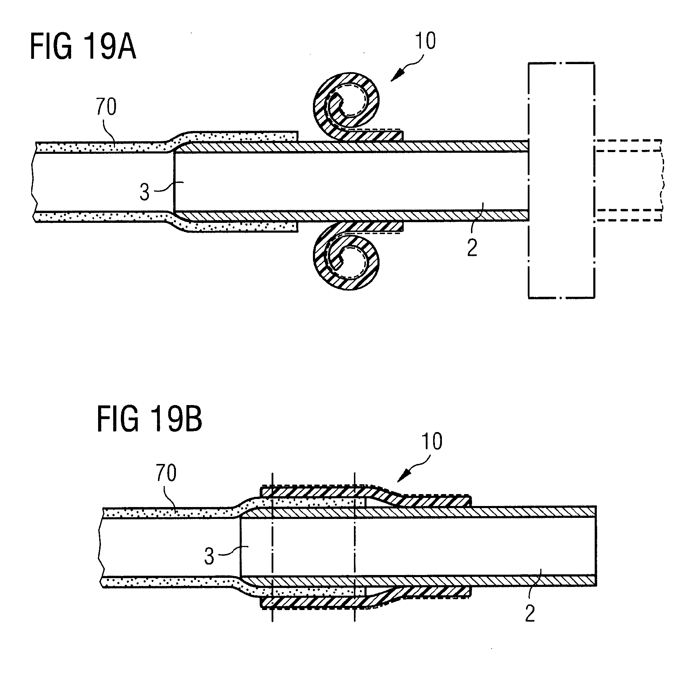

Figures 1 to 4, 6 to 11, 15 and 16 relate to the lateral connection and figures 19 to 21 relate to the cross-sectional sleeve connection, not the cross-sectional bulge and ring connection. However, for a complete understanding of the invention, these embodiments, albeit not falling within the scope of the claims, are maintained.

Figure 1 shows a system according to the present invention with an artificial intestine section 2 being implanted inside a patient's body 100 and having a first open end portion 3 connected to a surgically created opening in the patient's intestine 70, more specifically to a lateral opening in a wall of the patient's intestine 70. The second open end portion 4 exits the patient's abdominal wall 101 forming a stomy 170. The artificial intestine section 2 is here shown as a black box and includes at least one energy consuming part, such as one or more valves, a pump and/or any other flow control device, a motor for driving the same, possibly in connection with a reservoir. An accumulator A is implanted along with the artificial intestine section 2 and can be wirelessly charged from outside the patient's body 100. The energy E is here galvanically transmitted from the accumulator A to the artificial intestine section 2.

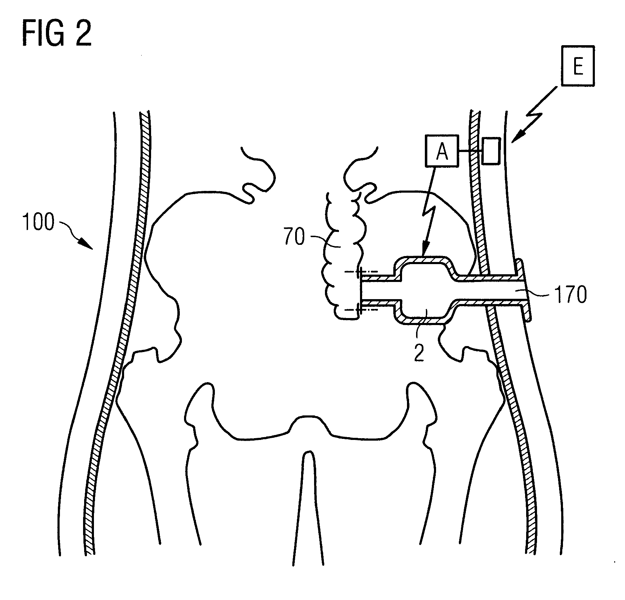

Figure 2 shows a system corresponding to the one shown in Figure 1, however, with the energy E being transmitted wirelessly from the accumulator A to the artificial intestine section 2.

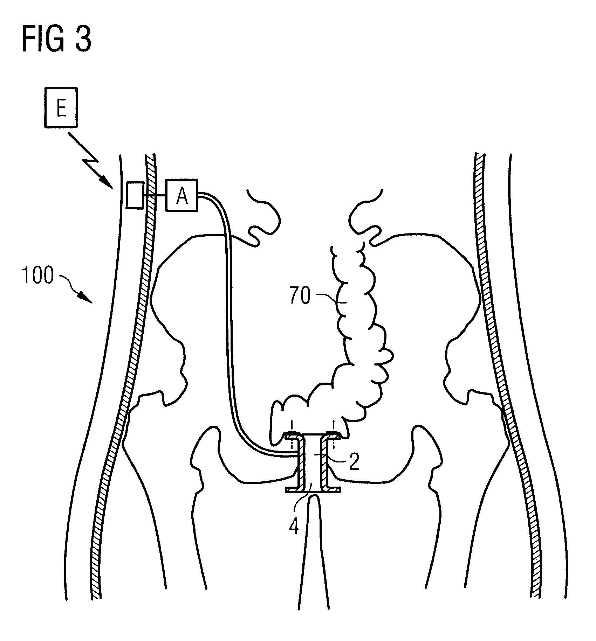

Figure 3 shows a system corresponding to the one shown in Figure 1, however, with the second open end portion 4 of the artificial intestine section 2 exiting the patient's anus.

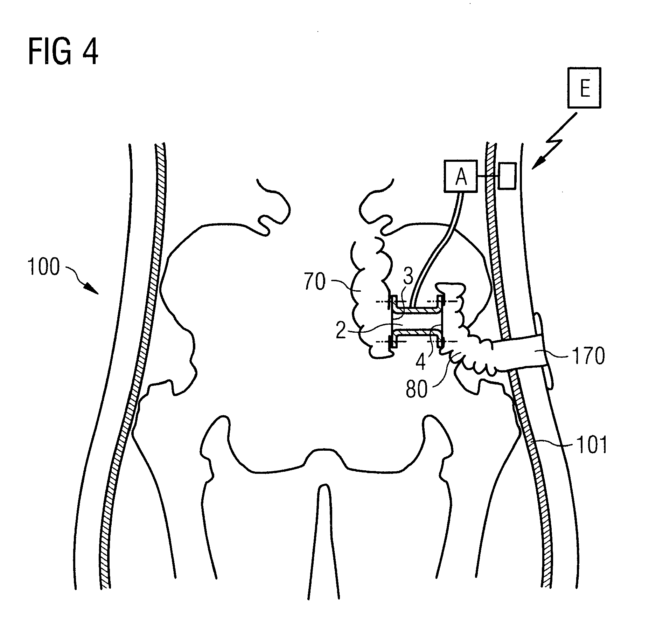

Figure 4 shows a system where both the first 3 and second 4 open end portions of the artificial intestine section 2 are attached to surgically created lateral openings in a wall of the patient's small 70 and/or large 80 intestine. The downstream part 80 of the intestine exits the patient's abdominal wall 101 forming a surgically created stomy 170. The downstream part 80 of the intestine may as well exit through the patient's anus.

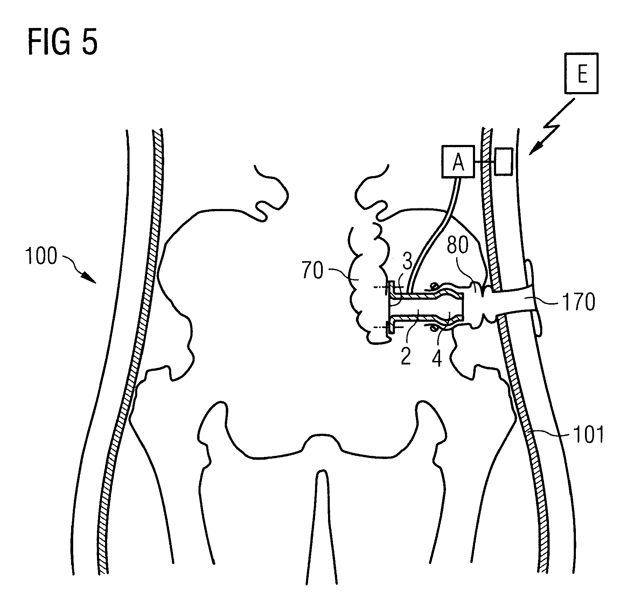

Figure 5 shows a similar system with the difference that the second open end portion 4 is connected to a cross-sectional opening of the patient's intestine 80, further leading to the surgically created stomy 170. The downstream part 80 of the intestine may as well exit through the patient's anus.

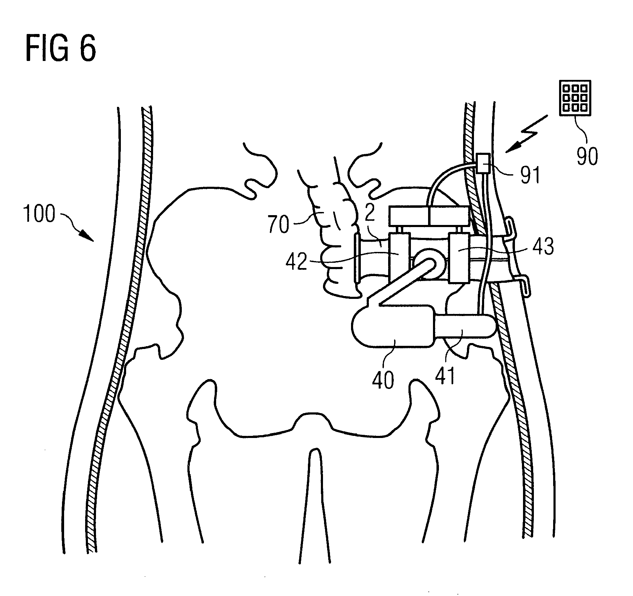

Figure 6 shows an embodiment of the artificial intestine section 2 with an artificial reservoir 40 and an entry valve 42 and exit valve 43 arranged upstream and downstream of the reservoir 40. The reservoir 40 is mounted with a pump 41 in a common housing and the pump 41 and the entry and exit valves 42, 43 are controlled by means of a control device, of which a part 91 is implanted inside the patient's body 100. Data are transmitted wirelessly between the external part 90 and implanted part 91 of the control unit. In addition, energy is wirelessly transmitted to the artificial intestine section 2 or to an accumulator also implanted in the patient's body 100 and galvanically connected here to the valves 42, 43 and pump 41.

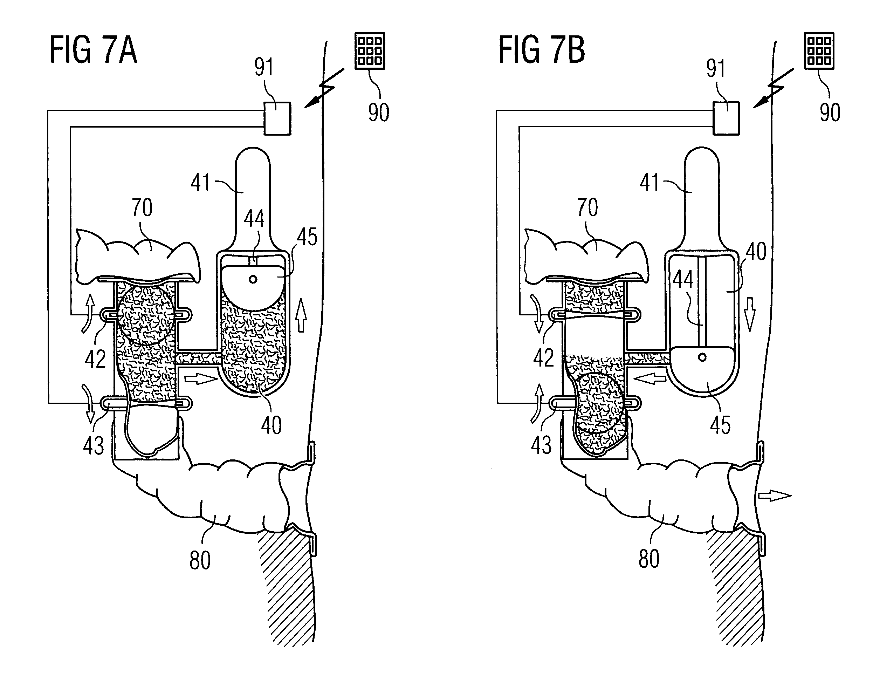

Figures 7A and 7B show a first embodiment of the structure of Figure 6 in more detail. The pump 41 comprises a moveable piston 44 with a front end 45 of the piston 44 extending into the reservoir 40 such that a volume of the reservoir 40 is reduced upon advancement of the piston 44. The piston 44 is spring loaded so as to urge the piston 44 into a normally retracted position. Furthermore, entry and exit valves 42 ,43 are here realized as flap valves. The flap valves are controlled so that one valve is open while the other one is closed.

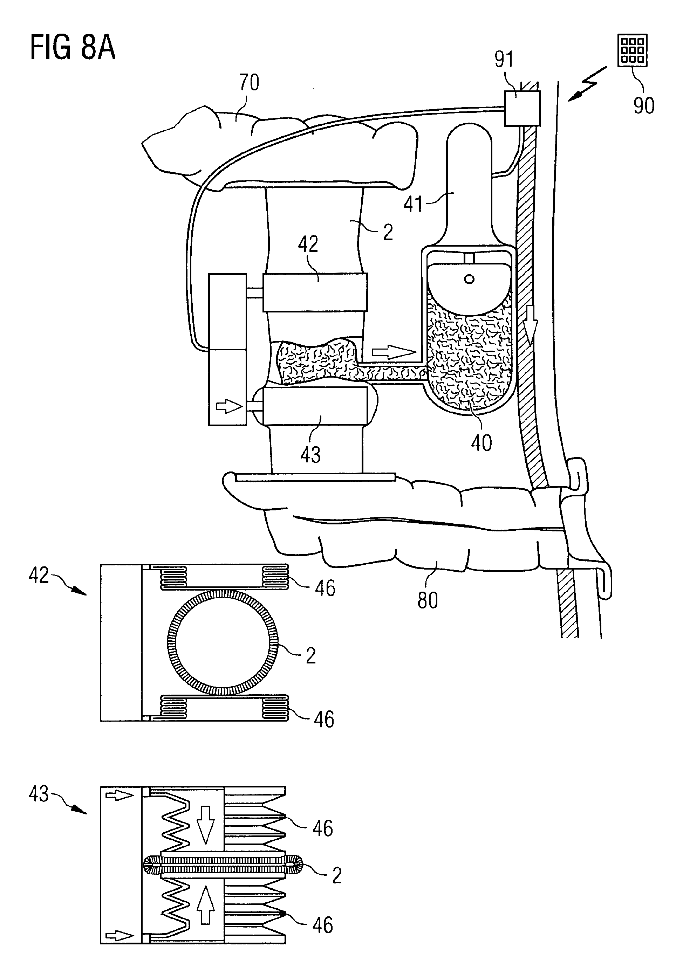

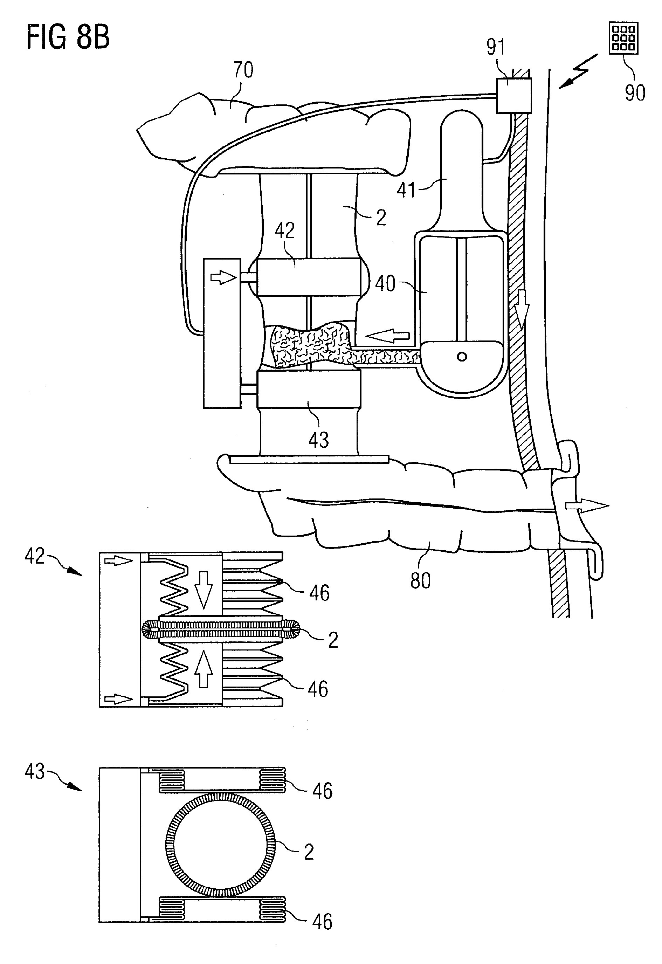

Figures 8A and 8B show a system similar to the one of Figures 7A and 7B. However, here the entry and exit valves 42, 43 comprise bellows 46 acting on the intestine from the outside so as to close the intestine by compression. In Figure 8A the bellows 46 of the exit valve 43 are expanded to compress the artificial intestine section 2 at the downstream side of the reservoir 40, whereas in Figure 8B the artificial intestine section 2 is closed by means of the bellows 46 of the entry valve 42 upstream of the reservoir 40 so that the reservoir 40 can be emptied by advancing the piston 44 of the pump 41.

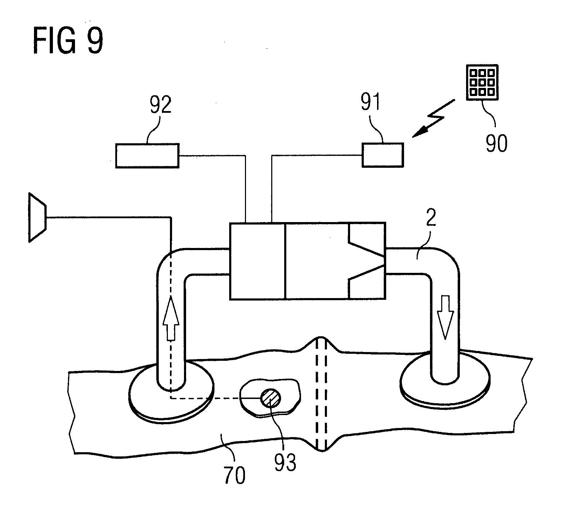

Figure 9 shows an embodiment schematically, wherein the artificial intestine section 2 by-passes a section of the patient's intestine 70, the intestine 70 being closed by sewing so as to direct intestinal content towards the artificial intestine section 2. The enlarged area of the artificial intestine section 2 represents any kind of element acting on the intestinal contents within the artificial intestine section, such as a reservoir, one or more valves, a pump or any other flow control device, possibly including a motor, and the like. Furthermore, a battery 92 implantable in the patient's body and preferably rechargeable provides the artificial intestine section 2 with energy. The artificial intestine section 2 is wirelessly controlled and the battery 92, if rechargeable, wirelessly charged. A sensor 93 implanted on or within the intestine 70 delivers data on the physical conditions within the intestine 70 for controlling the artificial intestine section 2.

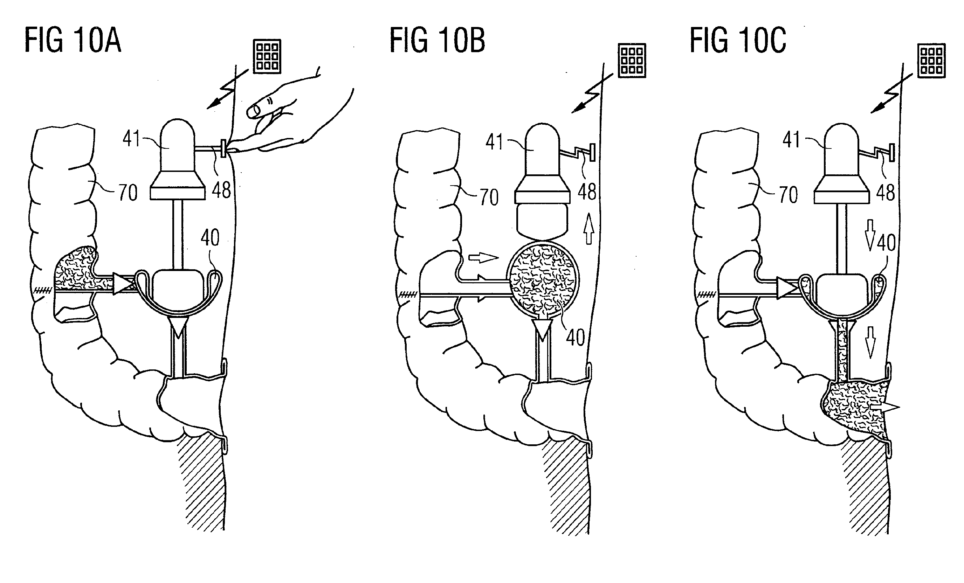

Figures 10A to 10C show an embodiment, where the artificial intestine section 2 comprises a reservoir 40 with a flexible wall. A pump 41 is implanted in the patient's body separate but in close proximity to the reservoir 40 and is used to empty the reservoir 40. The pump 41 is actuated by means of a subcutaneously implanted, manually operable switch 48.

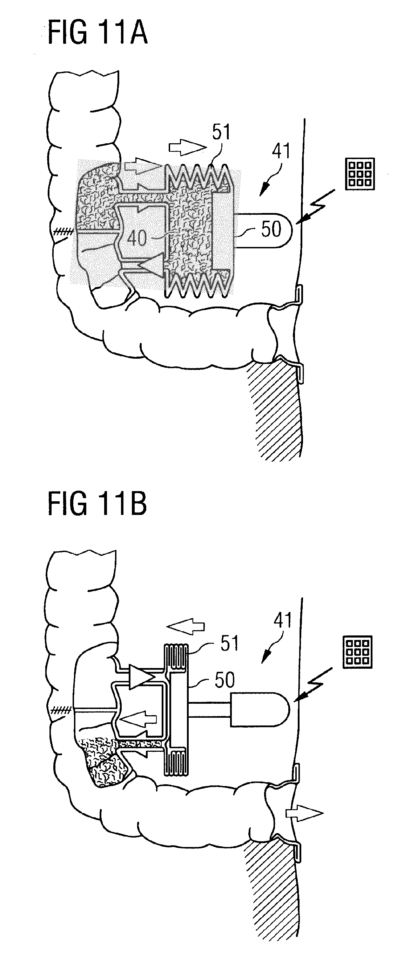

Figures 11 A and 11B show a structure similar to the one of Figures 10A to 10C, however, with the pump 41 and the reservoir 40 being fixedly connected to one another. The reservoir 40 is formed by a bellow 51 having an end wall 50 closing the bellow 51 at one end thereof. The end wall 50 makes part of the pump 41 such that a volume of the bellow 51 can be reduced upon advancement of the end wall 50. The bellow 51 is made of a resilient material so as to urge the bellow 51 into a normally extended position

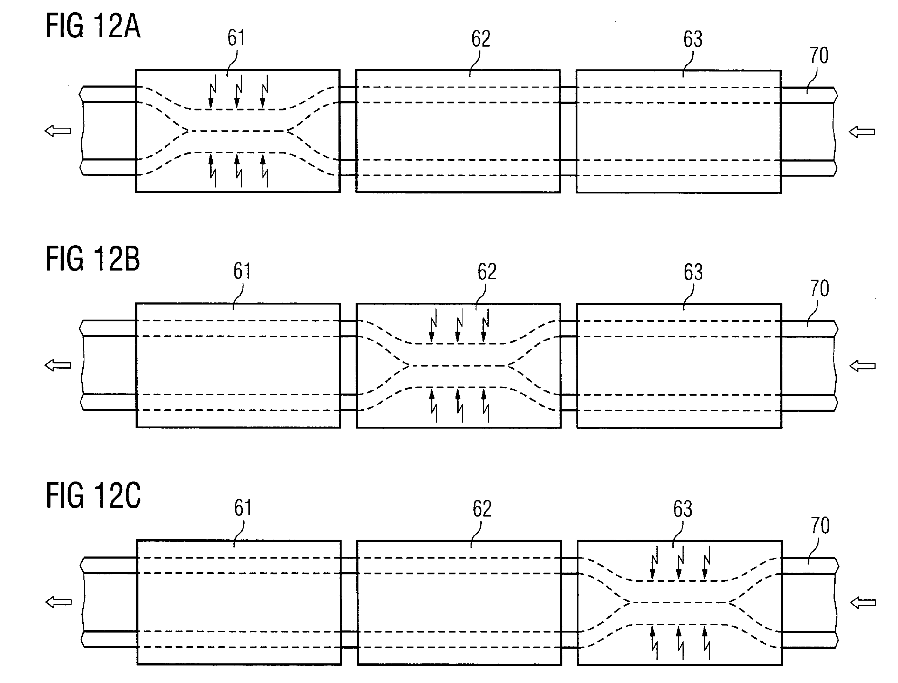

Figures 12A to 12C show a plurality of cooperating valves 61, 62, 63 implanted inside the patient's body and outside the patient's intestine 70. These can be positioned behind and/or in front of the artificial intestine piece along the patient's natural intestine 70. Each of the valves 61, 62, 63 comprises an electrical stimulation device adapted to electrically stimulate muscle or neural tissue of an intestine section so as to cause at least partial contraction of the intestine section. For that purpose, the stimulation device comprises at least one electrode adapted to apply electric pulses to the intestine section. While instead of the three stimulation devices shown, a single stimulation device would be sufficient for opening and closing the intestine, the arrangement of the plurality of stimulation devices is adapted to stimulate different portions of the intestine section over time. The function of the three stimulation devices may also be combined in one integral unit. The direction of natural intestinal contents flow is indicated by arrows. The different portions of the intestine section in a wavelike manner may be made in a direction opposite to the natural intestinal contents flow, as shown in Figures 12A to 12C, so as to close the intestine section. The stimulation in the wavelike manner may also be made in the direction of natural intestinal contents flow to support emptying of the intestine or reservoir.

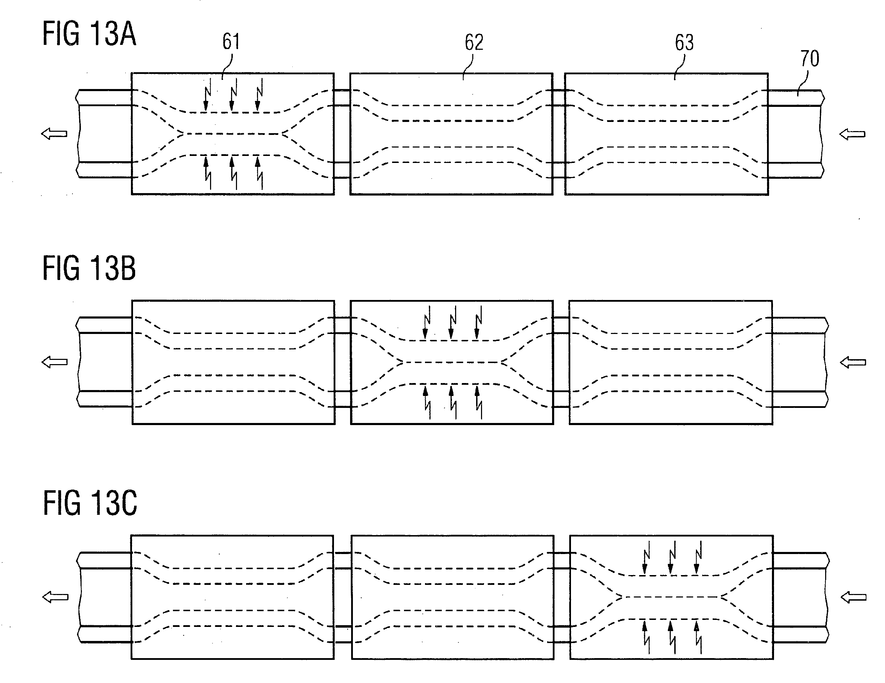

Figures 13A to 13C show the stimulation devices of Figures 12A to 12C in combination with constriction devices, such as the bellow valves described in relation to Figures 8A and 8B, for at least partly constricting the intestine section mechanically. Complete constriction is obtained by additional electrical stimulation of the respective intestine sections. The constriction devices may be released in order to allow intestinal contents to flow through.

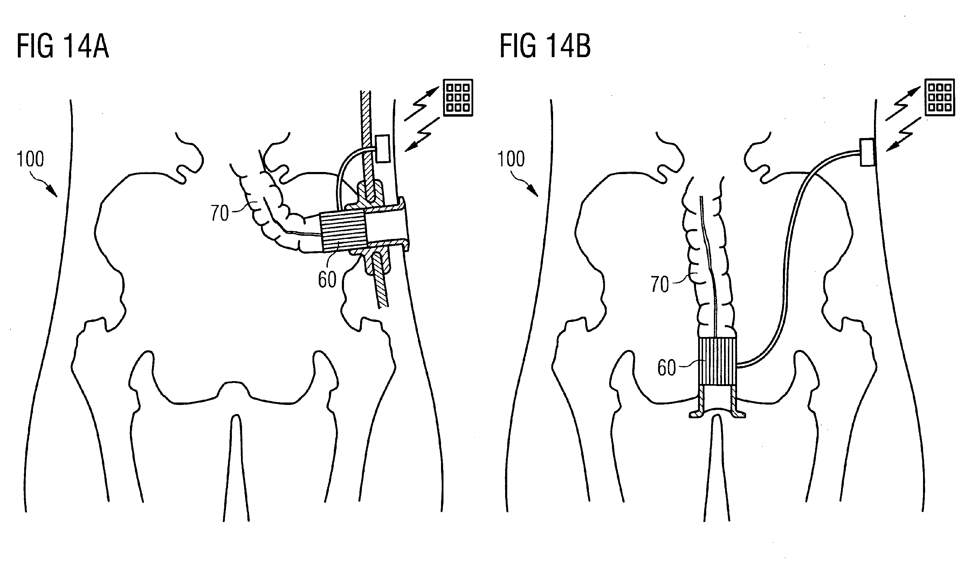

Figure 14A and 14B show a system comprising the artificial intestine section 2 connected to a cross-sectional opening of the patient's intestine 70 and having a valve 60 as shown in Figures 12 or 13 arranged around the patient's intestine 70 upstream of the artificial intestine section 2. Energy and/or data is transmitted wirelessly.

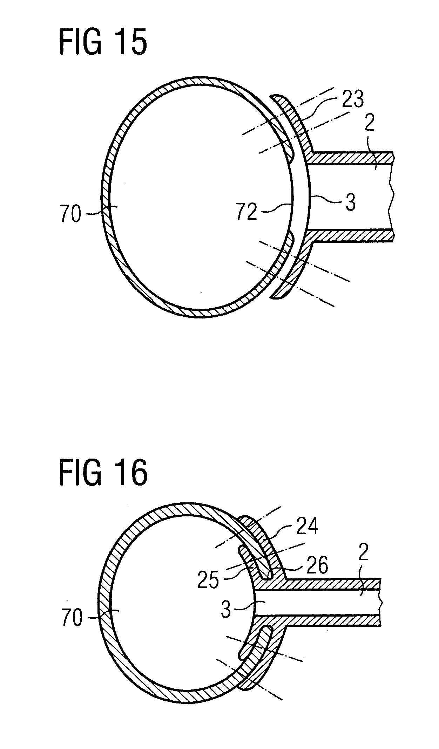

Figure 15 shows the structure of an open end portion 3 of the artificial intestine section 2 for attaching the artificial intestine section 2 to a lateral opening 72 in the patient's intestine 70 by means of a shoulder portion 23 formed around the end portion 3. The end portion 3 is sewn to the intestine 70 and may additionally or alternatively be stapled and/or glued to the intestine 70.

Figure 16 shows an improved structure for lateral attachment to the intestine 70, wherein the shoulder portion is split into an upper 24 and a lower 25 shoulder portion forming a gap 26 to accommodate intestinal wall tissue therein. The surface area of the upper shoulder portion 24 is larger than the surface area of the lower shoulder portion 25.

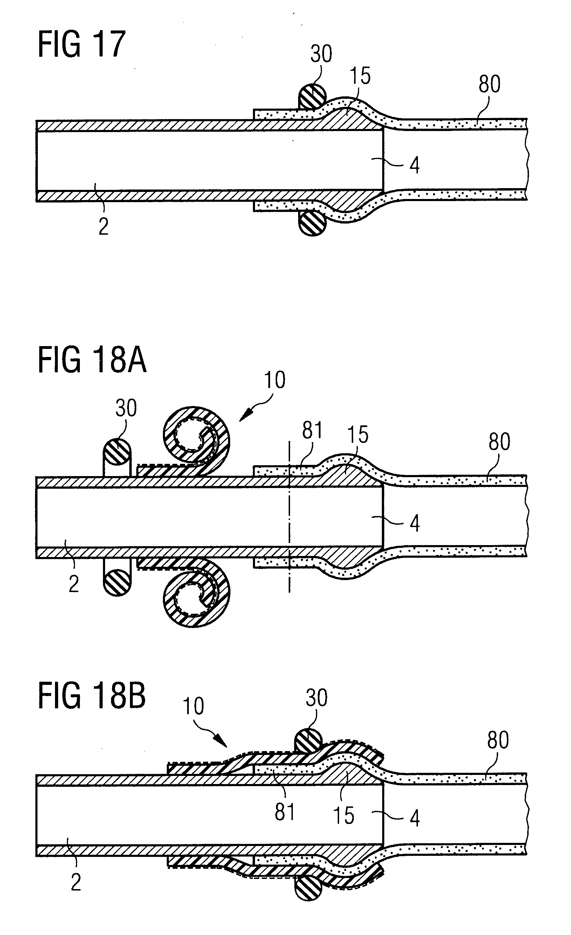

Figure 17 shows an enlarged view of a ring-and-bulge connection 15, 30 by which the artificial intestine section 2 and the patient's downstream intestinal part 80 are connected, as shown in Figure 5.

Figures 18A and 18B show the ring-and-bulge connection 15, 30 of Figure 17 in combination with a sleeve 10. The sleeve 10 is rolled upon itself and can be unrolled such that a part 80 of the intestine is located intermediate the sleeve 10 and the conduit 2. Thereafter, the ring 30 is pushed over the sleeve 10 against the bulge 15.

Figures 19A and 19B show a connection of the artificial intestine section 2 to a cross-sectional opening of the patient's intestine 70 similar to the connection shown in Figures 18A and 18B, however, without the bulge and the ring.

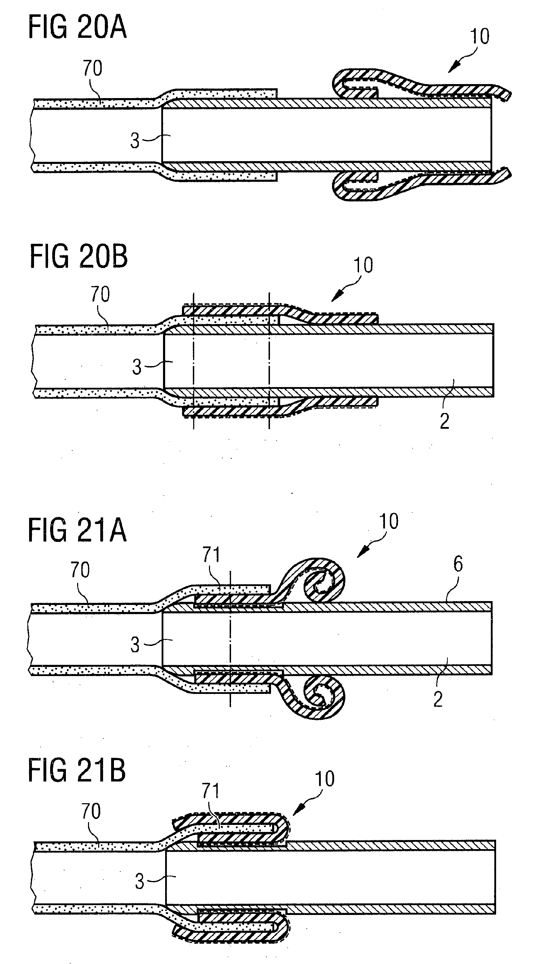

Figures 20A and 20B show an alternative to the connection in Figure 19A and 19B. Instead of unrolling the sleeve 10, it is simply pulled over the intestine 70.

Figures 21A and 21B show another sleeve connection. Here, the sleeve 10 is mounted on the outer surface 6 of the open end portion so as to be foldable upon itself. By folding the flexible sleeve 10 upon itself, a part 71 of the intestine 70 is located intermediate the folded sleeve 10.

Preferred embodiments of the invention are defined in paragraphs 1 to 186.

### ARTIFICIAL INTESTINE SECTION WITH WIRELESSLY CHARGED ACCUMULATOR AND BULGE STRUCTURE FOR FRONTAL ATTACHMENT TO INTESTINE

1. A system comprising an artificial intestine section adapted to being implanted inside a patient's body along with an accumulator for accumulating energy,
   - wherein said intestine section has a first open end portion and a second open end portion in flow communication with one another, at least the first open end portion being adapted to being connected to a surgically created opening in the patient's intestine, and
   - wherein said accumulator is adapted to be charged wirelessly with energy and arranged to supply energy directly or indirectly to at least one energy consuming part of said artificial intestine section,
   - wherein at least the first open end portion is adapted to being connected to a cross-sectional opening surgically created in the patient's intestine,
   - wherein at least the first open end portion comprises
   - a conduit having an outer surface with at least one bulge extending outwardly from the conduit's outer surface in a circumferential direction of the conduit about at least a part of the conduit's circumference, and
   - a blocking ring loosely fitting over the outer surface of the conduit with a clearance between the outer surface and the blocking ring for mounting intestinal tissue within the clearance, said blocking ring having an inner cross sectional diameter which is smaller than or substantially identical to an outer cross sectional diameter of the at least one bulge so as to prevent the blocking ring from slipping over the bulge when intestinal tissue is mounted within the clearance.

### LATERAL CONNECTION TO INTESTINE

2. The system of para 1, wherein the second open end portion is adapted to being connected to a lateral opening surgically created in a wall of the patient's intestine.

### STRUCTURE OF LATERAL ATTACHMENT

3. The system of para 2, wherein the second open end portion comprises a shoulder portion formed around the end portion for lateral connection to the patient's intestinal wall.
4. The system of para 3, wherein at least a part of the shoulder portion extends laterally from the artificial intestine section by 3 mm to 20 mm.
5. The system of any of paras 3 to 4, wherein the shoulder portion has a curved cross section, so as to generally conform to an intestinal wall when laterally attached thereto.
6. The system of any of paras 3 to 5, wherein the shoulder portion is split into an upper and a lower shoulder portion with a gap between the upper and lower shoulder portions adapted to accommodate intestinal wall tissue therein.
7. The system of para 6, wherein the lower shoulder portion is adapted to being placed inside the patient's intestine through a surgically created lateral opening in the intestinal wall and wherein the upper shoulder portion is adapted to being placed outside the intestinal wall.
8. The system of para 7, wherein the upper shoulder portion and the lower shoulder portion each have a surface area facing the intestinal wall, with the surface area of the upper shoulder portion being larger than the surface area of the lower shoulder portion.
9. The system of any of paras 3 to 8, wherein the shoulder portion is adapted to being connected to the patient's intestinal wall by gluing.
10. The system of any of paras 3 to 9, wherein the shoulder portion is adapted to being connected to the patient's intestinal wall by sewing.
11. The system of any of paras 3 to 10, wherein the shoulder portion is adapted to being connected to the patient's intestinal wall by stapling.
12. The system of any of paras 3 to 11, wherein the shoulder portion comprises a multilayer material.
13. The system of any of paras 3 to 12, wherein a surface to be facing the patient's intestinal wall has a porous in-growth layer allowing in-growth of living tissue.
14. The system of any of paras 3 to 13, wherein the shoulder portion comprises at least one biocompatible material selected from the following group of materials: titanium, stainless steel, ceramics, biocompatible polymer, other biocompatible polymer material.
15. The system of para 14, wherein the biocompatible polymer material comprises at least one polymer of the following group of polymers: polytetrafluoroethylene, silicone, polyurethane, expanded polytetrafluoroethylene (ePTFE).

### SLEEVE STRUCTURE FOR FRONTAL ATTACHMENT TO INTESTINE

16. The system of any one of paras 1 to 15, wherein at least the first open end portion comprises a flexible sleeve adapted to axially extend and closely fit around at least part of said outer surface of the conduit.
17. The system of any one of paras 1 to 16, wherein the second open end portion comprises
   - a conduit having an outer surface and
   - a flexible sleeve adapted to axially extend and closely fit around at least part of said outer surface of the conduit.
18. The system of para 16 or 17, wherein said flexible sleeve is mounted on said outer surface either folded or rolled upon itself or so as to be foldable upon itself.
19. The system of any of paras 1 to 18, wherein at least the first open end portion comprises a multilayer material.
20. The system of any of paras 1 to 19, wherein at least the first open end portion comprises a porous ingrowth layer allowing ingrowth of living tissue.
21. The system of para 20, wherein the ingrowth layer has a net-like structure.
22. The system of any of paras 20 to 21, wherein the ingrowth layer is made from Dacron®.

### THROUGH-FLOW ARRANGEMENT (STRAIGHT)

23. The system of any of paras 1 and 16 to 22, wherein both the first and second open end portions are adapted to being connected to a surgically created cross-sectional opening in the patient's intestine, so as to form an intermediate intestine section.

### DOWNSTREAM CONNECTION TO RESIDUAL INTESTINE

24. The system of any of paras 1 and 16 to 22, wherein the second open end portion is adapted to being connected to a portion of a patient's small intestine.
25. The system of any of paras 1 and 16 to 22, wherein the second open end portion is adapted to being connected to a portion of a patient's large intestine.

### STOMA CONNECTION

26. The system of any of paras 1 and 16 to 22, wherein the second open end portion is adapted to being connected to an artificial stoma, so as to form an intestine end section.

### ANUS CONNECTION

27. The system of any of paras 1 and 16 to 22, wherein the second open end portion is adapted to being connected to the patient's rectum or anus or to tissue adjacent the patient's anus, so as to form an intestine end section.

### MATERIAL

28. The system of any of paras 1 to 27, wherein at least the first open end portion is made from a biocompatible material.
29. The system of para 28, wherein the biocompatible material of the open end portion comprises at least one material of the following group of materials: titanium, stainless steel, ceramics, biocompatible polymer material, other polymer material.
30. The system of para 29, wherein the biocompatible polymer material comprises at least one polymer of the following group of polymers: polytetrafluoroethylene, silicone, polyurethane, expanded polytetrafluoroethylene (ePTFE).

### INTEST. CONTENT INTERACTING DEVICE AS AN ENERGY CONSUMING PART

31. The system of any of paras 1 to 30, wherein said at least one energy consuming part of the artificial intestine section comprises at least one element adapted to directly or indirectly interact with intestinal contents contained in the artificial intestine section between the first and second open end portions thereof.

### FLOW CONTROL DEVICE AS AN ENERGY CONSUMING PART

32. The system of para 31, wherein the energy consuming part comprises a flow control device adapted to control flow of intestinal contents from the artificial intestine section through the second open end portion.
33. The system of para 32, wherein the flow control device is adapted to prevent flow of intestinal contents from the artificial intestine section through the second open end portion.

### EXIT VALVE AS FLOW CONTROL DEVICE

34. The system of any of paras 32 to 33, wherein the flow control device comprises at least one valve, including an exit valve preventing intestinal contents flow through the second open end portion in its closed position.
35. The system of para 34, wherein the exit valve is a normally closed valve.

### ENTRY VALVE AS AN ADDITITONAL PART OF THE FLOW CONTROL DEVICE

36. The system of any of paras 32 to 35, wherein the flow control device comprises an entry valve allowing intestinal contents to flow through the first open end portion into the artificial intestine section in its open position.
37. The system of para 36, wherein the entry valve is a normally open valve.
38. The system of any of paras 36 to 37, wherein the exit valve and the entry valve are adapted to cooperate such that when one of the two valves is closed, the other valve is open, and vice versa.

### VALVE TYPES

39. The system of any of paras 34 to 38, wherein the at least one valve comprises a compartment with a variable volume adapted to open and close the valve by changing the compartment's volume.
40. The system of para 39, wherein the at least one valve comprises at least one passage for filling and emptying the compartment with hydraulic fluid.
41. The system of any of paras 39 to 40, wherein the compartment has at least one flexible wall defining an opening, the opening being adapted to close upon increase of the compartment's volume.
42. The system of any of paras 34 to 38, wherein the at least one valve is a flap valve.
43. The system of para 42, wherein the flap valve comprises a rotatable disc.

### EXTRA VALVE SEPARATE FROM ARTIFICIAL INTESTINE PIECE

44. The system of any of paras 1 to 43, further comprising at least one extra valve adapted to control flow of intestinal contents in a natural section of a patient's intestine upstream and/or downstream the artificial intestine section, wherein the extra valve is adapted to being implanted inside the patient's body outside a section of the patient's natural intestine and comprises at least one element adapted to act on the natural intestine section from the outside thereof so as to prevent intestinal contents flow through the natural intestine section.
45. The system of para 44, wherein the extra valve comprises at least one electrical stimulation device adapted to electrically stimulate muscle or neural tissue of the natural intestine section so as to cause at least partial contraction of the natural intestine section.
46. The system of para 45, wherein the stimulation device comprises at least one electrode adapted to apply electric pulses to the natural intestine section.
47. The system of any of paras 45 to 46, wherein the stimulation device is adapted to stimulate different portions of the natural intestine section over time.
48. The system of para 47, wherein the stimulation device is adapted to stimulate, over time, the different portions of the natural intestine section in a wave like manner in a direction opposite to natural intestinal contents flow.
49. The system of any of paras 44 to 48, wherein the extra valve comprises a constriction device for at least partly constricting the natural intestine section mechanically.
50. The system of para 49, including para 45, wherein the stimulation device is combined with the constriction device so that the stimulation device and the constriction device act on the same natural intestine section.
51. The system of para 50, wherein the constriction device in its normal condition constricts the natural intestine section only partly.
52. The system of any of paras 50 to 51, wherein the stimulation device is adapted to pump intestinal contents along the natural intestine section by, over time, stimulating the different portions of the natural intestine section in a wave like manner in a direction of natural intestinal contents flow.

### PUMP AS AN ENERGY CONSUMING PART

53. The system of any of paras 31 to 52, wherein said at least one energy consuming part of the artificial intestine section comprises a pump for advancing intestinal contents through the second open end portion to outside the artificial intestine section.

### PUMP WITH RESERVOIR

54. The system of para 53, wherein the artificial intestine section comprises a reservoir between the first and second open end portions for receiving and temporarily collecting therein intestinal contents supplied through the first open end portion, and wherein the pump is adapted for emptying the reservoir through the second open end portion.
55. The system of para 54, wherein the reservoir is formed by a bellow, said bellow having an end wall closing the bellow at one end thereof, said end wall making part of the pump such that a volume of the bellow is reduced upon advancement of said end wall.
56. The system of para 55, wherein the bellow is made of a resilient material so as to urge the bellow into a normally expanded position.
57. The system of para 54, wherein the pump comprises a movable piston, with a front end of the piston extending into the reservoir such that a volume of the reservoir is reduced upon advancement of the piston.
58. The system of para 57, wherein the piston is spring loaded so as to urge the piston into a normally retracted position.
59. The system of para 54, wherein the pump is adapted for being permanently arranged inside the reservoir.
60. The system of para 54, wherein the reservoir has a flexible wall and the pump is adapted for emptying the reservoir by squeezing the reservoir.
61. The system of para 60, wherein the pump includes a constriction device adapted to alternately constrict and release sections of the reservoir so as to pump intestinal contents along the reservoir by, over time, constricting different sections of the reservoir in a wave like manner.
62. The system of para 61, wherein the reservoir has a tube-like form and the pump is a roller pump acting on the tube-like reservoir from the outside thereof.

### MOTOR AS AN ENERGY CONSUMING PART

63. The system of any of paras 1 to 62, wherein said at least one energy consuming part of the artificial intestine section comprises at least one motor arranged for automatically driving at least one further energy consuming part of the artificial intestine section.
64. The system of para 63, including the artificial intestine section of any of paras 34 to 52, wherein the at least one motor is arranged for driving at least one of the valve or valves, respectively, between closed and open positions.
65. The system of any of paras 63 to 64, including para 53, wherein the at least one motor is arranged for driving the pump.
66. The system of any of paras 58 to 60, comprising a manually operable switch for activating the at least one motor, the switch being arranged for subcutaneous implantation so as to be operable from outside the patient's body.
67. The system of any of paras 63 to 66, wherein the motor is arranged to be driven by electric or electromagnetic energy.

### ENERGY TRANSMISSION

68. The system of any of paras 1 to 67, comprising galvanic coupling elements between the accumulator and the energy consuming part for transmitting energy to the energy consuming part in contacting fashion.
69. The system of any of paras 1 to 67, comprising an implantable wireless energy transmitter and an implantable wireless energy receiving device for wirelessly transmitting energy from the accumulator to the energy consuming part.
70. The system of para 69, wherein the energy consuming part is adapted to directly transform the wirelessly transmitted energy from the accumulator into kinetic energy.
71. The system of para 69, wherein the energy consuming part comprises a transforming device for transforming the wirelessly transmitted energy from the accumulator into electric energy.
72. The system of any of paras 1 to 71, comprising an implantable energy transforming device for transforming the wirelessly transmitted energy from outside the patient's body into energy to be stored in the accumulator and further comprising a wireless energy transmitter adapted to wirelessly transmit energy from outside the patient's body to said implantable energy transforming device.
73. The system of para 72, wherein the implantable energy transforming device transforms wireless energy into electric energy and the energy consuming part is adapted to be driven with the electric energy, as said energy transforming device transforms the wireless energy into the electric energy.
74. The system of any of paras 72 to 73, wherein the energy transmitter is adapted to generate an electromagnetic field.
75. The system of any of paras 72 to 74, wherein the energy transmitter is adapted to generate a magnetic field.
76. The system of any of paras 72 to 75, wherein the energy transmitter is adapted to generate an electrical field.
77. The system of any of paras 72 to 76, wherein the energy transmitter is adapted to transmit energy by at least one wireless energy signal.
78. The system of para 77, wherein the wireless energy signal comprises an electromagnetic wave signal, including at least one of an infrared light signal, a visible light signal, an ultra violet light signal, a laser signal, a microwave signal, an X-ray radiation signal, and a gamma radiation signal.
79. The system of para 78, wherein the wireless energy signal comprises a sound or ultrasound wave signal.
80. The system of any of paras 77 to 79, wherein the wireless energy signal comprises a digital or analog signal or a combination thereof.

### ENERGY TRANSMISSION FEEDBACK

81. The system of any of paras 72 to 80, comprising a feedback subsystem adapted to wirelessly send feedback information related to the energy to be stored in the accumulator from inside the human body to the outside thereof, wherein the system is adapted to use the feedback information for adjusting the amount of wireless energy transmitted by the energy transmitter.
82. The system of para 81, wherein the feedback information is related to an energy balance which is defined as the balance between an amount of wireless energy received inside the human body and an amount of energy consumed by the at least one energy consuming part.
83. The system of para 82, wherein the feedback information is related to an energy balance which is defined as the balance between a rate of wireless energy received inside the human body and a rate of energy consumed by the at least one energy consuming part.

### ACCUMULATOR

84. The system of any of paras 1 to 83, wherein the accumulator comprises a rechargeable battery.
85. The system of any of paras 1 to 84, wherein the accumulator comprises a capacitor.
86. The system of any of paras 1 to 85, wherein the accumulator is adapted for being implanted inside the patient's body fixedly connected to the artificial intestine section.
87. The system of any of paras 1 to 85, wherein the accumulator is adapted for being implanted inside the patient's body distant to the artificial intestine section.

### PRIMARY ENERGY SOURCE

88. The system of any of paras 1 to 87, comprising a primary energy source for charging the accumulator with energy from outside the patient's body.
89. The system of para 88, wherein the primary energy source is adapted to being mounted on the patient's body.

### CONTROL UNIT

90. The system of any of paras 1 to 89, further comprising a control unit adapted to directly or indirectly control one or more elements of the system.
91. The system of para 90, including para 34, wherein the control unit is adapted to control opening of the exit valve.
92. The system of any of paras 90 to 91, including para 36, wherein the control unit is adapted to control closing of the entry valve.
93. The system of para 92, wherein the control unit is adapted to control opening of the exit valve and closing of the entry valve such that when one of the two valves is closed, the other valve is open, and vice versa.
94. The system of any of paras 90 to 93, including para 53, wherein the control unit is adapted to control actuation of the pump.
95. The system of any of paras 90 to 94, wherein the control unit is operable by the patient.
96. The system of any of paras 90 to 95, wherein at least part of the control unit is implantable in the patient's body.
97. The system of para 96, comprising a manually operable switch for activating the control unit, the switch being arranged for subcutaneous implantation so as to be operable from outside the patient's body.
98. The system of any of paras 90 to 97, wherein the control unit comprises a first part adapted for implantation in the patient's body and a second part adapted to cooperate with the first part from outside the patient's body.
99. The system of para 98, including para 72, wherein the control unit is adapted to transmit data from the second part of the control unit to the implantable first part of the control unit in the same manner as energy is transmitted by said wireless energy transmitter from outside the patient's body to said implantable energy transforming device.
100. The system of any of paras 98 to 99, wherein the second part of the control unit is adapted to wirelessly transmit a control signal to the implantable first part of the control unit for controlling the at least one energy consuming part from outside the patient's body.
101. The system of any of paras 98 to 100, wherein the implantable first part of the control unit is programmable via the second part of the control unit.
102. The system of any of paras 98 to 100, wherein the implantable first part of the control unit is adapted to transmit a feedback signal to the second part of the control unit.

### SENSOR

103. The system of any of paras 1 to 102, further comprising a physical parameter sensor adapted to directly or indirectly sense a physical parameter of the patient.
104. The system of para 103, wherein the physical parameter sensor is adapted to sense at least one of the following physical parameters of the patient: a pressure within the artificial intestine section, a pressure within the patient's natural intestine, an expansion of the artificial intestine section, a distension of an intestinal wall of the patient's natural intestine, a movement of the patient's intestinal wall.
105. The system of any of paras 1 to 104, further comprising a functional parameter sensor adapted to directly or indirectly sense a functional parameter of the system.
106. The system of para 105, wherein the functional parameter sensor is adapted to sense at least one of the following functional parameters of the system: a pressure against a part of the system such as the artificial intestine section, a distension of a part of the system such as a wall of the artificial intestine section, an electrical parameter such as voltage, current or energy balance, a position or movement of a movable part of the system.
107. The system of any of paras 105 to 106, comprising an indicator coupled to the sensor, the indicator being adapted to provide a signal when the sensor senses a value for the parameter beyond a predetermined threshold value.
108. The system of para 107, wherein the signal comprises at least one of the following types of signals: a sound signal, a visual signal.

### METHOD OF TREATMENT (IMPLANTATION)

109. A surgical method of treating a patient, comprising the steps of:
   - cutting the patient's skin and abdominal wall,
   - dissecting an area of the patient's intestine,
   - surgically creating at least one opening in the dissected intestinal area so as to create an artificial intestinal opening,
   - providing the artificial intestine section of the system of any of paras 1 to 108 and affixing its first open end portion to the artificial intestinal opening so as to be in flow communication therewith,
   - arranging the accumulator of said system inside the patient's body so as to allow for supplying energy directly or indirectly from the accumulator to at least one energy consuming part of said artificial intestine section, and
   - suturing the abdominal wall and skin.
110. A laparoscopic surgical method of treating a patient, comprising the steps of:
   - making a small opening in the patient's skin and abdominal wall,
   - introducing a needle in the abdominal cavity,
   - inflating the abdominal cavity with gas,
   - inserting at least one trocar into the cavity,
   - introducing a camera through the trocar,
   - inserting at least one dissecting instrument preferably through a second trocar,
   - dissecting an area of the intestine,
   - surgically creating at least one opening in the dissected intestinal area so as to create an artificial intestinal opening,
   - providing the artificial intestine section of the system of any of paras 1 to 108 and affixing its first open end portion to the artificial intestinal opening so as to be in flow communication therewith,
   - arranging the accumulator of said system inside the patient's body so as to allow for supplying energy directly or indirectly from the accumulator to at least one energy consuming part of said artificial intestine section, and
   - extracting the instruments, camera and trocar, and in relation thereto
   - suturing, if necessary, the abdominal wall and permanently closing the skin.
111. The method of any of paras 109 to 110, wherein the accumulator is provided fixedly connected to the artificial intestine section and is arranged inside the patient's body along with the artificial intestine section.
112. The method of any of paras 109 to 110, wherein the accumulator is provided separate from the artificial intestine section and is arranged inside the patient's body distant to the artificial intestine section.
113. The method of para 112, comprising the step of galvanically coupling the accumulator to the artificial intestine section for transmitting energy to the at least one energy consuming part of the artificial intestine section in contacting fashion.

### DIVIDING THE INTESTINE

114. The method of any of paras 109 to 113, wherein the step of surgically creating at least one opening in the dissected intestinal area comprises the steps of:
   - dissecting a portion of the dissected intestinal area downstream of the intestinal opening to be created, such that intestinal mesentery connected to the dissected portion is opened in such a way that supply of blood through the mesentery to the dissected intestinal area is maintained as far as possible on both sides of the dissected portion, and
   - dividing the patient's intestine in the dissected portion so as to create an upstream part of the intestine with an artificial intestinal opening being formed as a cross-sectional opening at the downstream side thereof and a downstream part of the intestine with an artificial intestinal opening being formed as a cross-sectional opening at the upstream side thereof, wherein the mesentery maintains a tissue connection between the upstream and downstream intestine parts.

### FRONTAL CONNECTION AT UPSTREAM END

115. The method of para 114, comprising the step of affixing the first open end portion of the artificial intestine section to the cross-sectional downstream opening of the upstream intestine part so as to be in flow communication therewith.

### FRONTAL CONNECTION AT DOWNSTREAM END

116. The method of para 114, comprising the steps of affixing the first open end portion of the artificial intestine section to the cross-sectional upstream opening of the downstream intestine part so as to be in flow communication therewith.

### FRONTAL CONNECTION AT BOTH ENDS

117. The method of para 114, comprising the steps of
   - affixing one end of the first and second open end portions of the artificial intestine section to the cross-sectional downstream opening of the upstream intestine part so as to be in flow communication therewith and
   - affixing the other end of the first and second open end portions of the artificial intestine section to the cross-sectional upstream opening of the downstream intestine part so as to be in flow communication therewith.

### FRONTAL CONNECTION UPSTREAM, LATERAL CONNECTION DOWNSTREAM

118. The method of para 114, comprising the steps of
   - closing the cross-sectional opening at the downstream side of the upstream intestine part,
   - surgically creating an opening in a wall of the upstream intestine part so as to create a lateral intestinal opening therein,
   - affixing the second open end portion of the artificial intestine section to the lateral intestinal opening of the upstream intestine part so as to be in flow communication therewith and
   - affixing the first open end portion of the artificial intestine section to the cross-sectional upstream opening of the downstream intestine part so as to be in flow communication therewith.

### SLEEVE/BULGE CONNECTOR FOR FRONTAL CONNECTION

119. The method of any of paras 115 to 118, wherein the step of affixing the open end portion of the artificial intestine section to the cross-sectional opening of the intestine part comprises:
   - inserting the open end portion of the artificial intestine section into the cross-sectional opening of the intestine part, and
   - placing the flexible sleeve so as to extend over both the intestine part and open end portion of the artificial intestine section such that the intestine part is located intermediate the sleeve and the outer surface of the artificial intestine section.
120. The method of para 119, wherein the flexible sleeve is mounted on the outer surface of the open end portion of the artificial intestine piece so as to be foldable upon itself and wherein the step of placing the flexible sleeve so as to extend over both the intestine part and open end portion of the artificial intestine section comprises folding the flexible sleeve upon itself such that the intestine part is located intermediate the folded sleeve.
121. The method of any of paras 115 to 118, wherein the step of affixing the open end portion of the artificial intestine section to the cross-sectional opening of the intestine part comprises:
   - inserting the artificial intestine section having the bulge formed on the outside thereof into the cross-sectional opening of the intestine part so that the intestine part extends over the bulge from one side of the bulge, and
   - advancing the blocking ring over the intestine part towards the bulge from the respective other side of the bulge such that the intestine part is located intermediate the outer surface of the artificial intestine section and the blocking ring.

### LATERAL CONNECTION UPSTREAM, FRONTAL CONNECTION DOWNSTREAM

122. The method of para 114, comprising the steps of
   - closing the cross-sectional opening at the upstream side of the downstream intestine part,
   - surgically creating an opening in a wall of the downstream intestine part so as to create a lateral intestinal opening therein,
   - affixing the second open end portion of the artificial intestine section to the lateral intestinal opening of the downstream intestine part so as to be in flow communication therewith and
   - affixing the first open end portion of the artificial intestine section to the cross-sectional downstream opening of the upstream intestine part so as to be in flow communication therewith.

### STRUCTURE OF LATERAL ATTACHMENT

123. The method of para 118 or 122, wherein the step of affixing the open end portion of the artificial intestine section to the lateral intestinal opening so as to be in flow communication therewith comprises connecting a shoulder portion, which is formed around the open end portion of the artificial intestine section, to the patient's intestinal wall so as to surround the lateral intestinal opening.
124. The method of para 123, wherein the step of connecting the shoulder portion comprises attaching the shoulder portion to the patient's outer intestinal wall.
125. The method of para 123, wherein the shoulder portion is split into an upper and a lower shoulder portion with a gap between the upper and lower shoulder portions, and wherein the step of affixing the open end portion of the artificial intestine section to the lateral intestinal opening comprises placing the lower shoulder portion inside the patient's intestine and the upper shoulder portion outside the patient's intestine such that intestinal wall tissue is accommodated in the gap.
126. The method of any of paras 123 to 125, wherein the step of affixing the open end portion of the artificial intestine section to the lateral intestinal opening comprises gluing the open end portion to the patient's intestinal wall.
127. The method of any of paras 123 to 126, wherein the step of affixing the open end portion of the artificial intestine section to the lateral intestinal opening comprises sewing the open end portion to the patient's intestinal wall.
128. The method of any of paras 123 to 127, wherein the step of affixing the open end portion of the artificial intestine section to the lateral intestinal opening comprises stapling the open end portion to the patient's intestinal wall.

### EXIT THROUGH STOMA

129. The method of any of paras 109 to 128, further comprising the steps of:
   - cutting the patient's skin and abdominal wall so as to create an opening for an intestinal stomy,
   - dissecting the area of the stomy opening,
   - dividing the intestine at a location downstream of the artificial intestine section so as to create an upstream natural intestine section having a cross-sectional opening at the downstream end thereof and a downstream natural intestine section,
   - dissecting the mesentery of the upstream natural intestine section in the area of the cross-sectional opening thereof to prepare for creating the intestinal stomy,
   - advancing the downstream end of the upstream natural intestine section through the abdominal wall and skin, and
   - suturing the cross-sectional opening of the upstream natural intestine section to the skin with the intestinal mucosa turned inside out, thereby achieving the intestinal stomy.

### EXIT THROUGH ANUS

130. The method of any of paras 109 to 128, further comprising the steps of:
   - dividing the intestine at a location downstream of the artificial intestine section so as to create an upstream natural intestine section having a cross-sectional opening at the downstream end thereof and a downstream natural intestine section leading to the patient's anus,
   - dissecting the area of the patient's anus and surgically separating the downstream natural intestine section from the patient's anus, whereas the steps of dividing the intestine and separating the intestine section leading to the patient's anus can alternatively be carried out in reversed order,
   - dissecting the mesentery of the upstream natural intestine section in the area of the cross-sectional opening at the downstream end thereof to prepare for connecting the upstream natural intestine section to the patient's anus or tissue adjacent the patient's anus,
   - advancing the downstream end of the upstream natural intestine section through the patient's anus, and
   - suturing the cross-sectional opening of the upstream natural intestine section to the patient's anus or tissue adjacent the patient's anus.
131. The method of any of paras 129 to 130, wherein the natural intestine section is selected from the patient's small intestine.
132. The method of any of paras 129 to 130, wherein the natural intestine section is selected from the patient's large intestine.

### INTEST. CONTENT INTERACTING DEVICE AS AN ENERGY CONSUMING PART

133. The method of any of paras 109 to 132, comprising the step of implanting along with the artificial intestine section at least one energy consuming part adapted to directly or indirectly interact with intestinal contents contained in the artificial intestine section between the first and second open end portions thereof.

### FLOW CONTROL DEVICE

134. The method of para 133, wherein the energy consuming part comprises a flow control device adapted to control flow of intestinal contents from the artificial intestine section through the second open end portion.

### EXIT VALVE

135. The method of para 134, wherein the flow control device comprises an exit valve preventing intestinal contents flow through the second open end portion in its closed position.

### ENTRY VALVE

136. The method of any of paras 134 to 135, wherein the flow control device comprises an entry valve allowing intestinal contents to flow through the first open end portion into the artificial intestine section in its open position.

### PUMP

137. The method of any of paras 134 to 136, wherein the flow control device comprises a pump for advancing intestinal contents through the second open end portion to outside the artificial intestine section.

### PUMP WITH RESERVOIR

138. The method of para 137, wherein the at least one element comprises a reservoir between the first and second open end portions for receiving and temporarily collecting therein intestinal contents supplied through the first open end portion, the pump being adapted to empty the reservoir through the second open end portion.
139. The method of any of paras 137 to 138, wherein the pump comprises a manually operable switch for activating the pump, the method further comprising the step of implanting the switch subcutaneously so as to be operable from outside the patient's body.

### MOTOR

140. The method of any of paras 109 to 139, wherein the at least one energy consuming part comprises at least one motor arranged for automatically driving at least one further energy consuming part of the artificial intestine section.
141. The method of para 140, wherein the motor comprises a manually operable switch for activating the motor, the method further comprising the step of implanting the switch subcutaneously so as to be operable from outside the patient's body.

### ENERGY TRANSMISSION

142. The method of any of paras 109 to 141, further comprising the step of implanting an energy transforming device for transforming wireless energy into energy to be stored in the accumulator.

### ACCUMULATOR

143. The method of any of paras 109 to 142, wherein at least one rechargeable battery is provided as the accumulator.
144. The method of any of paras 109 to 143, wherein at least one capacitor is provided as the accumulator.

### CONTROL UNIT

145. The method of any of paras 110 to 144, further comprising the step of implanting inside the patient's body at least a part of a control unit adapted to directly or indirectly control at least one element implanted in the patient's body.
146. The method of para 145, wherein the control unit comprises a manually operable switch for activating the control unit, the method further comprising the step of implanting said switch subcutaneously so as to be operable from outside the patient's body.

### SENSOR

147. The method of any of paras 109 to 146, further comprising the step of implanting a physical parameter sensor adapted to directly or indirectly sense a physical parameter inside the patient.
148. The method of any of paras 109 to 147, further comprising the step of implanting a functional parameter sensor adapted to directly or indirectly sense a functional parameter of the system inside the patient.
149. The method of any of paras 147 to 148, wherein the sensor is a pressure sensor and is placed in the artificial intestine section or the patient's natural intestine so as to sense the pressure within the artificial intestine section or patient's natural intestine, respectively.
150. The method of any of paras 147 to 149, wherein the sensor is a tension sensor and is placed in contact with the artificial intestine section or the patient's natural intestine so as to sense an expansion of the artificial intestine section or patient's natural intestine, respectively.
151. The method of any of paras 147 to 150, wherein the sensor is a movement sensor and is placed in contact with the artificial intestine section or the patient's natural intestine so as to sense movement of the artificial intestine section or patient's natural intestine, respectively.
152. The method of any of paras 148 to 151, wherein the sensor is adapted to measure at least one of the following functional parameters: an electrical parameter such as voltage, current or energy balance or a stimulation parameter in relation to the system.

### USE

153. A method of treating a patient by means of the system of para 31, comprising the step of actuating the at least one element of the energy consuming part of the system's artificial intestine section so as to interact with intestinal contents contained in the artificial intestine section between the first and second open end portions of the artificial intestine section.

### EXIT AND ENTRY VALVE

154. The method of treating a patient according to para 153, wherein the at least one element comprises an exit valve preventing intestinal contents flow from the artificial intestine section through the second open end portion in its closed position, the method further comprising the steps of opening the exit valve and then removing intestinal contents from the artificial intestine section.
155. The method of treating a patient according to para 154, wherein the at least one element comprises an entry valve allowing intestinal contents to flow through the first open end portion into the artificial intestine section in its open position, the method further comprising the step of closing the entry valve before removing intestinal contents from the artificial intestine section.

### PUMP

156. The method of any of paras 153 to 155, wherein the at least one element comprises a pump, the method further comprising the step of advancing intestinal contents from the artificial intestine section through the second open end portion thereof to outside the artificial intestine section by means of the pump.

### PUMP WITH RESERVOIR

157. The method of para 156, wherein the artificial intestine section comprises a reservoir between the first and second open end portions for receiving and temporarily collecting therein intestinal contents supplied through the first open end portion, and wherein the step of advancing intestinal contents to outside the intestine section comprises operating the pump so as to empty the reservoir.
158. The method of para 157, wherein the reservoir is formed by a bellow, said bellow having an end wall closing the bellow at one end thereof and said end wall making part of the pump, wherein the step of advancing intestinal contents to outside the intestine section comprises advancing the end wall so as to reduce a volume of the bellow.
159. The method of para 157, wherein the pump comprises a movable piston with a front end of the piston extending into the reservoir, wherein the step of advancing intestinal contents to outside the intestine section comprises advancing the piston so as to reduce a volume of the reservoir.
160. The method of para 157, wherein the reservoir has a flexible wall and wherein the step of advancing intestinal contents to outside the intestine section comprises squeezing the reservoir by means of the pump.
161. The method of para 160, wherein the pump includes a constriction device and wherein the step of advancing intestinal contents to outside the intestine section comprises alternately constricting and releasing sections of the reservoir so as to pump intestinal contents along the reservoir by, over time, constricting different sections of the reservoir in a wave like manner.
162. The method of para 161, wherein the reservoir has a tube-like form and the pump is a roller pump acting on the tube-like reservoir from the outside thereof.

### MOTOR

163. The method of any of paras 154 to 155, wherein the at least one element comprises a motor, the method further comprising the step of driving at least the exit valve between its closed and open positions by means of the motor.
164. The method of any of paras 156 to 162, wherein the at least one element comprises a motor, the method further comprising the step of driving at least the pump by means of the motor.
165. The method of any of paras 163 to 164, further comprising the step of activating the motor by manually operating a subcutaneously arranged actuator from outside the patient's body.

### ENERGY

166. The method of any of paras 153 to 165, further comprising the steps of:
   - transmitting energy wirelessly from outside the patient's body,
   - transforming the wirelessly transmitted energy into energy to be stored in the accumulator by means of an energy transforming device implanted in the patient's body, and
   - charging the accumulator with at least part of the transformed energy.
167. The method of para 166, further comprising the step of supplying energy from the accumulator to at least one implanted energy consuming part.
168. The method of para 167, wherein in the step of supplying the energy from the accumulator to the energy consuming part, the energy is transmitted wirelessly.
169. The method of any of paras 166 to 168, comprising the step of transforming at least part of the wirelessly transmitted energy into electric energy and supplying said part of transformed energy to the energy consuming part, as said part of the wirelessly transmitted energy is transformed into the electric energy.
170. The method of any of paras 166 to 169, wherein the wireless energy is transmitted by means of an electromagnetic field.
171. The method of any of paras 166 to 170, wherein the wireless energy is transmitted by means of a magnetic field.
172. The method of any of paras 166 to 171, wherein the wireless energy is transmitted by means of an electrical field.
173. The method of any of paras 166 to 172, wherein the wireless energy is transmitted by at least one wireless energy signal.
174. The method of para 173, wherein the wireless energy signal comprises an electromagnetic wave signal, including at least one of an infrared light signal, a visible light signal, an ultra violet light signal, a laser signal, a microwave signal, an X-ray radiation signal, and a gamma radiation signal.
175. The method of para 173, wherein the wireless energy signal comprises a sound or ultrasound wave signal.
176. The method of any of paras 173 to 175, wherein the wireless energy signal comprises a digital or analog signal or a combination thereof.

### CONTROL

177. The method of any of paras 153 to 176, wherein a first part of a control unit for controlling at least one energy consuming part of the artificial intestine section is implanted inside the patient's body, the method further comprising the step of using an external second part of the control unit adapted to cooperate with the first part from outside the patient's body to transmit data to the implanted first part of the control unit.
178. The method of para 177, including para 166, wherein the data are transmitted to the implanted first part of the control unit in the same manner as energy is transmitted wirelessly for charging the accumulator.
179. The method of any of paras 177 to 178, comprising the step of programming the implanted first part of the control unit via the external second part of the control unit.
180. The method of any of paras 177 to 179, comprising the step of transmitting a feedback signal from the implanted first part of the control unit to the external second part of the control unit.

### SENSOR

181. The method of any of paras 153 to 180, comprising the step of sensing a physical parameter in the patient's body.
182. The method of para 181, wherein the step of sensing a physical parameter in the patient's body comprises sensing at least one of the following physical parameters of the patient: a pressure within the artificial intestine section, a pressure within the patient's natural intestine, an expansion of the artificial intestine section, a distension of an intestinal wall of the patient's natural intestine, a movement of the patient's intestinal wall.
183. The method of any of paras 153 to 182, comprising the step of sensing a functional parameter of the system in the patient's body.
184. The method of para 183, wherein the step of sensing a functional parameter of the system in the patient's body comprises sensing at least one of the following parameters: a pressure against a part of the system such as the artificial intestine section, a distension of a part of the system such as a wall of the artificial intestine section, an electrical parameter such as voltage, current or energy balance, a position or movement of a movable part of the system.
185. The method of any of paras 181 to 184, comprising the step of providing a signal when a value for the physical parameter sensed is beyond a predetermined threshold value.
186. The method of para 185, wherein the step of providing a signal includes at least one of the following types of signals: a sound signal, a visual signal.

## Claims

1. A system comprising an artificial intestine section (2) adapted to being implanted inside a patient's body (100) along with an accumulator (A) for accumulating energy (E),
- wherein said intestine section (2) has a first open end portion (3) and a second open end portion (4) in flow communication with one another, at least the first open end portion (3) being adapted to being connected to a surgically created opening in the patient's intestine (70, 80),
- wherein said accumulator (A) is adapted to be charged wirelessly with energy and arranged to supply energy (E) directly or indirectly to at least one energy consuming part of said artificial intestine section (2), and
- wherein at least the first open end portion (3) is adapted to being connected to a cross-sectional opening surgically created in the patient's intestine (70, 80),
**characterized in that** at least the first open end portion (3) comprises
- a conduit having an outer surface with at least one bulge (15) extending outwardly from the conduit's outer surface in a circumferential direction of the conduit about at least a part of the conduit's circumference, and
- a blocking ring (30) loosely fitting over the outer surface of the conduit with a clearance between the outer surface and the blocking ring (30) for mounting intestinal tissue within the clearance, said blocking ring (30) having an inner cross sectional diameter which is smaller than or substantially identical to an outer cross sectional diameter of the at least one bulge (15) so as to prevent the blocking ring (30) from slipping over the bulge (15) when intestinal tissue is mounted within the clearance.

2. The system of claim 1, wherein at least the first open end portion (3) comprises a flexible sleeve (10) adapted to axially extend and closely fit around at least part of said outer surface of the conduit.

3. The system of claim 2, wherein said flexible sleeve (10) is mounted on said outer surface either folded or rolled upon itself or so as to be foldable upon itself.

4. The system of any of claims 1 to 3, wherein at least the first open end portion (3) comprises a multilayer material.

5. The system of any of claims 1 to 4, wherein at least the first open end portion (3) comprises a porous ingrowth layer allowing ingrowth of living tissue.

6. The system of any of claims 1 to 5, wherein both the first and second open end portions (3, 4) are adapted to being connected to a surgically created cross-sectional opening in the patient's intestine (70, 80), so as to form an intermediate intestine section (2).

7. The system of any of claims 1 to 6, wherein the energy (E) consuming part comprises an exit valve (43) preventing intestinal contents flow through the second open end portion (4) in its closed position, wherein the exit valve (43) is preferably a normally closed valve.

8. The system of any of claims 1 to 7, wherein the energy (E) consuming part comprises an entry valve (42) allowing intestinal contents to flow through the first open end portion (3) into the artificial intestine section (2) in its open position, wherein the entry valve (42) is preferably a normally open valve.

9. The system of claims 7 and 8, wherein the exit valve (43) and the entry valve (42) are adapted to cooperate such that when one of the two valves (42, 43) is closed, the other valve is open, and vice versa.

10. The system of any of claims 1 to 9, wherein said at least one energy (E) consuming part of the artificial intestine section (2) comprises a pump (41) for advancing intestinal contents through the second open end portion (4) to outside the artificial intestine section (2).

11. The system of claim 10, wherein the artificial intestine section (2) comprises a reservoir (40) between the first and second open end portions (3, 4) for receiving and temporarily collecting therein intestinal contents supplied through the first open end portion (3), and wherein the pump (41) is adapted for emptying the reservoir (40) through the second open end portion (4).

12. The system of claim 11, wherein the reservoir (40) is formed by a bellow (51), said bellow (51) having an end wall closing the bellow (51) at one end thereof, said end wall making part of the pump (41) such that a volume of the bellow (51) is reduced upon advancement of said end wall, wherein the bellow (51) is preferably made of a resilient material so as to urge the bellow (51) into a normally expanded position.

13. The system of claim 11, wherein the pump (41) comprises a movable piston (44), with a front end (45) of the piston (44) extending into the reservoir (40) such that a volume of the reservoir (40) is reduced upon advancement of the piston (44), wherein the piston (44) is preferably spring loaded so as to urge the piston (44) into a normally retracted position.

14. The system of any of claims 1 to 13, wherein said at least one energy consuming part of the artificial intestine section (2) comprises at least one motor arranged for automatically driving at least one further energy consuming part of the artificial intestine section (2).

15. The system of claim 14, comprising a manually operable switch for activating the at least one motor, the switch being arranged for subcutaneous implantation so as to be operable from outside the patient's body (100).

16. The system of any of claims 1 to 15, comprising an implantable energy transforming device for transforming wirelessly transmitted energy from outside the patient's body (100) into energy to be stored in the accumulator (A) and further comprising a wireless energy transmitter adapted to wirelessly transmit energy from outside the patient's body (100) to said implantable energy transforming device.

17. The system of claim 16, comprising a feedback subsystem adapted to wirelessly send feedback information related to the energy to be stored in the accumulator (A) from inside the human body (100) to the outside thereof, wherein the system is adapted to use the feedback information for adjusting the amount of wireless energy transmitted by the energy transmitter.

18. The system of claim 17, wherein the feedback information is related to an energy balance which is defined as the balance between an amount of wireless energy received inside the human body (100) and an amount of energy consumed by the at least one energy consuming part and/or wherein the feedback information is related to an energy balance which is defined as the balance between a rate of wireless energy received inside the human body (100) and a rate of energy consumed by the at least one energy consuming part.

19. The system of any of claims 1 to 18, further comprising a control unit (90, 91) adapted to directly or indirectly control one or more elements of the system, wherein the control unit (90, 91) comprises a first part (91) adapted for implantation in the patient's body (100) and a second part (90) adapted to cooperate with the first part (91) from outside the patient's body (100).

20. The system of any of claims 1 to 19, further comprising a physical parameter sensor (93) adapted to directly or indirectly sense a physical parameter of the patient, wherein the physical parameter sensor (93) is preferably adapted to sense at least one of the following physical parameters of the patient: a pressure within the artificial intestine section (2), a pressure within the patient's natural intestine (70, 80), an expansion of the artificial intestine section (2), a distension of an intestinal wall of the patient's natural intestine (70, 80), a movement of the patient's intestinal wall, and/or comprising a functional parameter sensor adapted to directly or indirectly sense a functional parameter of the system, wherein the functional parameter sensor is preferably adapted to sense at least one of the following functional parameters of the system: a pressure against a part of the system such as the artificial intestine section (2), a distension of a part of the system such as a wall of the artificial intestine section (2), an electrical parameter such as voltage, current or energy balance, a position or movement of a movable part of the system.

## Patentansprüche

1. System, umfassend einen künstlichen Darmabschnitt (2), der angepasst ist, in einen Körper (100) eines Patienten zusammen mit einem Akkumulator (A) zum Akkumulieren von Energie (E) implantiert zu werden,
- wobei der Darmabschnitt (2) einen ersten offenen Endteil (3) und einen zweiten offenen Endteil (4) in Flusskommunikation miteinander aufweist, wobei zumindest der erste offene Endteil (3) angepasst ist, mit einer chirurgisch hergestellten Öffnung im Darm (70, 80) des Patienten verbunden zu werden,
- wobei der Akkumulator (A) angepasst ist, drahtlos mit Energie geladen zu werden, und eingerichtet ist, Energie (E) direkt oder indirekt zu zumindest einem Energie verbrauchenden Teil des künstlichen Darmabschnitts (2) zu liefern, und
- wobei zumindest der erste offene Endteil (3) angepasst ist, mit einer im Darm (70, 80) des Patienten chirurgisch hergestellten Querschnittsöffnung verbunden zu werden,
**dadurch gekennzeichnet, dass** zumindest der erste offene Endteil (3) umfasst:
- eine Leitung, die eine äußere Oberfläche mit zumindest einer Ausbuchtung (15) aufweist, die sich nach außen von der äußeren Oberfläche der Leitung in einer Umfangsrichtung der Leitung um zumindest einen Teil des Umfangs der Leitung erstreckt, und
- einen Blockierring (30), der lose über die äußere Oberfläche der Leitung passt, mit einer Lücke zwischen der äußeren Oberfläche und dem Blockierring (30) zum Anbringen von Darmgewebe in der Lücke, wobei der Blockierring (30) einen inneren Querschnittsdurchmesser aufweist, welcher kleiner oder im Wesentlichen identisch zu einem äußeren Querschnittsdurchmesser der zumindest einen Ausbuchtung (15) ist, um zu verhindern, dass der Blockierring (30) über die Ausbuchtung (15) rutscht, wenn Darmgewebe in der Lücke angebracht ist.

2. System nach Anspruch 1, wobei zumindest der erste offene Endteil (3) eine flexible Manschette (10) umfasst, die angepasst ist, sich axial um zumindest einen Teil der äußeren Oberfläche der Leitung herum zu erstrecken und darum eng zu passen.

3. System nach Anspruch 2, wobei die flexible Manschette (10) auf der äußeren Oberfläche entweder gefaltet oder über sich selbst aufgerollt oder um über sich selbst faltbar zu sein angebracht ist.

4. System nach einem der Ansprüche 3, wobei zumindest der erste offene Endteil (3) ein Mehrschichtmaterial umfasst.

5. System nach einem der Ansprüche 1 bis 4, wobei zumindest der erste offene Endteil (3) eine poröse Einwachsschicht umfasst, die ein Einwachsen von lebendem Gewebe erlaubt.

6. System nach einem der Ansprüche 1 bis 5, wobei sowohl der erste als auch der zweite offene Endteil (3, 4) angepasst sind, mit einer chirurgisch hergestellten Querschnittsöffnung im Darm (70, 80) des Patienten verbunden zu werden, um einen Zwischendarmabschnitt (2) zu bilden.

7. System nach einem der Ansprüche 1 bis 6, wobei der Energie (E) verbrauchende Teil ein Ausgangsventil (43) umfasst, das in seiner geschlossenen Position verhindert, dass Darminhalte durch den zweiten offenen Endteil (4) hindurch fließen, wobei das Ausgangsventil (43) vorzugsweise ein normalerweise geschlossenes Ventil ist.

8. System nach einem der Ansprüche 1 bis 7, wobei der Energie (E) verbrauchende Teil ein Eingangsventil (42) umfasst, das in seiner offenen Position erlaubt, dass Darminhalte durch den ersten offenen Endteil (3) hindurch in den künstlichen Darmabschnitt (2) hinein fließen, wobei das Eingangsventil (42) vorzugsweise ein normalerweise offenes Ventil ist.

9. System nach Anspruch 7 und 8, wobei das Ausgangsventil (43) und das Eingangsventil (42) angepasst sind, zusammenzuarbeiten, so dass, wenn eines der zwei Ventile (42, 43) geschlossen ist, das andere Ventil offen ist, und umgekehrt.

10. System nach einem der Ansprüche 1 bis 9, wobei der zumindest eine Energie (E) verbrauchende Teil des künstlichen Darmabschnitts (2) eine Pumpe (41) zum Fördern von Darminhalten durch den zweiten offenen Endteil (4) aus dem künstlichen Darmabschnitt (2) hinaus umfasst.

11. System nach Anspruch 10, wobei der künstliche Darmabschnitt (2) ein Reservoir (40) zwischen dem ersten und zweiten offenen Endteil (3, 4) zum Aufnehmen und temporären Sammeln von durch den ersten offenen Endteil (3) gelieferten Darminhalten darin umfasst, und wobei die Pumpe (41) zum Leeren des Reservoirs (40) durch den zweiten offenen Endteil (4) angepasst ist.

12. System nach Anspruch 11, wobei das Reservoir (40) durch einen Balg (51) gebildet ist, wobei der Balg (51) eine Endwand aufweist, die den Balg (51) an einem Ende davon schließt, wobei die Endwand einen Teil der Pumpe (41) ausmacht, so dass ein Volumen des Balgs (51) beim Vorschieben der Endwand reduziert wird, wobei der Balg (51) vorzugsweise aus einem federndem Material besteht, um den Balg (51) in eine normalerweise expandierte Position zu drängen.

13. System nach Anspruch 11, wobei die Pumpe (41) einen beweglichen Kolben (44) umfasst, wobei sich ein vorderes Ende (45) des Kolbens (44) in das Reservoir (40) hinein erstreckt, so dass ein Volumen des Reservoirs (40) beim Vorschieben des Kolbens (44) reduziert wird, wobei der Kolben (44) vorzugsweise federbelastet ist, um den Kolben (44) in eine normalerweise zurückgezogene Position zu drängen.

14. System nach einem der Ansprüche 1 bis 13, wobei der zumindest eine Energie verbrauchende Teil des künstlichen Darmabschnitts (2) zumindest einen Motor umfasst, der zum automatischen Antreiben zumindest eines weiteren Energie verbrauchenden Teils des künstlichen Darmabschnitts (2) eingerichtet ist.

15. System nach Anspruch 14, umfassend einen manuell betätigbaren Schalter zum Aktivieren des zumindest einen Motors, wobei der Schalter für subkutane Implantation eingerichtet ist, um von außerhalb des Körpers (100) des Patienten betätigbar zu sein.

16. System nach einem der Ansprüche 1 bis 15, umfassend eine implantierbare Energietransformationsvorrichtung zum Transformieren drahtlos übertragener Energie von außerhalb des Körpers (100) des Patienten in in dem Akkumulator (A) zu speichernde Energie, und des Weiteren umfassend einen drahtlosen Energieüberträger, der angepasst ist, Energie von außerhalb des Körpers (100) des Patienten zu der implantierbaren Energietransformationsvorrichtung drahtlos zu übertragen.

17. System nach Anspruch 16, umfassend ein Rückmeldungssubsystem, das angepasst ist, Rückmeldungsinformation bezüglich der in dem Akkumulator (A) zu speichernden Energie von innerhalb des menschlichen Körpers (100) nach außen drahtlos zu senden, wobei das System angepasst ist, die Rückmeldungsinformation zum Anpassen der Menge von von dem Energieüberträger übertragener drahtloser Energie zu verwenden.

18. System nach Anspruch 17, wobei die Rückmeldungsinformation mit einer Energiebilanz zusammenhängt, welche definiert ist als die Bilanz zwischen einer Menge von im menschlichen Körper (100) empfangener drahtloser Energie und einer Menge von Energie, die von dem zumindest einen Energie verbrauchenden Teil verbraucht wird, und/oder wobei die Rückmeldungsinformation mit einer Energiebilanz zusammenhängt, welche definiert ist als die Bilanz zwischen einer Rate von im menschlichen Körper (100) empfangener drahtloser Energie und einer Rate von Energie, die von dem zumindest einen Energie verbrauchenden Teil verbraucht wird.

19. System nach einem der Ansprüche 1 bis 18, des Weiteren umfassend eine Steuereinheit (90, 91), die angepasst ist, eines oder mehrere Elemente des Systems direkt oder indirekt zu steuern, wobei die Steuereinheit (90, 91) einen ersten Teil (91) umfasst, der zur Implantation in den Körper (100) des Patienten angepasst ist, und einen zweiten Teil (90), der angepasst ist, mit dem ersten Teil (91) von außerhalb des Körpers (100) des Patienten zusammenzuarbeiten.

20. System nach einem der Ansprüche 1 bis 19, des Weiteren umfassend einen Sensor (93) für physikalische Parameter, der angepasst ist, einen physikalischen Parameter des Patienten direkt oder indirekt zu erfassen, wobei der Sensor (93) für physikalische Parameter vorzugsweise angepasst ist, zumindest einen der folgenden physikalischen Parameter des Patienten zu erfassen: einen Druck innerhalb des künstlichen Darmabschnitts (2), einen Druck innerhalb des natürlichen Darms (70, 80) des Patienten, eine Ausdehnung des künstlichen Darmabschnitts (2), eine Dehnung einer Darmwand des natürlichen Darms (70, 80) des Patienten, eine Bewegung der Darmwand des Patienten, und/oder umfassend einen Sensor für funktionelle Parameter, der angepasst ist, einen funktionellen Parameter des Systems direkt oder indirekt zu erfassen, wobei der Sensor für funktionelle Parameter vorzugsweise angepasst ist, zumindest einen der folgenden funktionellen Parameter des Systems zu erfassen: einen Druck gegen einen Teil des Systems, wie den künstlichen Darmabschnitt (2), eine Dehnung eines Teils des Systems, wie einer Wand des künstlichen Darmabschnitts (2), einen elektrischen Parameter, wie Spannung, Stromstärke oder Energiebilanz, eine Position oder Bewegung eines beweglichen Teils des Systems.

## Revendications

1. Système comprenant une section d'intestin artificiel (2) apte à être implantée à l'intérieur d'un corps (100) de patient conjointement avec un accumulateur (A) destiné à accumuler de l'énergie (E) :
- dans lequel ladite section d'intestin (2) présente une première portion à extrémité ouverte (3) et une seconde portion à extrémité ouverte (4) permettant une communication d'écoulement entre l'une et l'autre, au moins la première portion à extrémité ouverte (3) étant apte à être reliée à une ouverture créée par voie chirurgicale dans l'intestin (70, 80) du patient ;
- dans lequel ledit accumulateur (A) est apte à être chargé en énergie de manière sans fil et est agencé pour fournir l'énergie (E) directement ou indirectement à au moins une partie consommant de l'énergie de ladite section d'intestin artificiel (2), et
- dans lequel au moins la première portion à extrémité ouverte (3) est apte à être reliée à une ouverture en travers créée par voie chirurgicale dans l'intestin (70, 80) du patient,
**caractérisé en ce qu'**au moins la première portion à extrémité ouverte (3) comprend :
- un conduit présentant une surface extérieure avec au moins un renflement (15) s'étendant vers l'extérieur à partir de la surface extérieure du conduit dans une direction circonférentielle du conduit autour d'au moins une partie de la circonférence du conduit, et
- un anneau de blocage (30) ajusté de manière libre sur la surface extérieure du conduit avec un espacement entre la surface extérieure et l'anneau de blocage (30) destiné à l'installation d'un tissu intestinal à l'intérieur de l'espacement, ledit anneau de blocage (30) présentant un diamètre de section intérieur qui est inférieur ou en grande partie identique à un diamètre de section extérieur du au moins un renflement (15), de manière à empêcher l'anneau de blocage (30) de glisser sur le renflement (15) lorsque le tissu intestinal est installé dans l'espacement.

2. Système selon la revendication 1, dans lequel au moins la première portion à extrémité ouverte (3) comprend un manchon flexible (10) apte à s'étendre de manière axiale et à présenter un ajustement serré autour d'au moins une partie de ladite surface extérieure du conduit.

3. Système selon la revendication 2, dans lequel ledit manchon flexible (10) est installé sur ladite surface extérieure, soit replié soit roulé sur lui-même ou de manière à pouvoir être replié sur lui-même.

4. Système selon l'une quelconque des revendications 1 à 3, dans lequel au moins la première portion à extrémité ouverte (3) comprend un matériau multicouche.

5. Système selon l'une quelconque des revendications 1 à 4, dans lequel au moins la première portion à extrémité ouverte (3) comprend une couche de repousse poreuse permettant la repousse de tissu vivant.

6. Système selon l'une quelconque des revendications 1 à 5, dans lequel les première et seconde portions à extrémité ouverte (3, 4) sont toutes deux aptes à être reliées à une ouverture en travers créée par voie chirurgicale dans l'intestin (70, 80) du patient de manière à former une section d'intestin intermédiaire (2).

7. Système selon l'une quelconque des revendications 1 à 6, dans lequel la partie consommant de l'énergie (E) comprend une valve de sortie (43) empêchant l'écoulement du contenu intestinal à travers la seconde portion à extrémité ouverte (4) dans sa position fermée, dans lequel la valve de sortie (43) est de préférence une valve normalement fermée.

8. Système selon l'une quelconque des revendications 1 à 7, dans lequel la partie consommant de l'énergie (E) comprend une valve d'entrée (42) permettant l'écoulement du contenu intestinal à travers la première portion à extrémité ouverte (3) vers la section d'intestin artificiel (2) dans sa position ouverte, dans lequel la valve d'entrée (42) est de préférence une valve normalement ouverte.

9. Système selon les revendications 7 et 8, dans lequel la valve de sortie (43) et la valve d'entrée (42) sont aptes à coopérer de telle sorte que lorsqu'une des deux valves (42, 43) est fermée, l'autre valve est ouverte, et inversement.

10. Système selon l'une quelconque des revendications 1 à 9, dans lequel ladite au moins une partie consommant de l'énergie (E) de la section d'intestin artificiel (2) comprend une pompe (41) destinée à faire avancer le contenu intestinal à travers la seconde portion à extrémité ouverte (4) vers l'extérieur de la section d'intestin artificiel (2).

11. Système selon la revendication 10, dans lequel la section d'intestin artificiel (2) comprend un réservoir (40) entre les première et seconde portions à extrémité ouverte (3, 4) destiné à recevoir et collecter temporairement à l'intérieur le contenu intestinal fourni à travers la première portion à extrémité ouverte (3), et dans lequel la pompe (41) est apte à vider le réservoir (40) à travers la seconde portion à extrémité ouverte (4).

12. Système selon la revendication 11, dans lequel le réservoir (40) est formé par un soufflet (51), ledit soufflet (51) présentant une paroi d'extrémité fermant le soufflet (51) sur une extrémité, ladite paroi d'extrémité faisant partie de la pompe (41), de telle sorte qu'un volume du soufflet (51) est réduit lors de l'avance de ladite paroi d'extrémité, dans lequel le soufflet (51) est de préférence fabriqué dans un matériau résilient de manière à pousser le soufflet (51) dans une position normalement dilatée.

13. Système selon la revendication 11, dans lequel la pompe (41) comprend un piston pouvant être déplacé (44), une extrémité avant (45) du piston (44) s'étendant jusque dans le réservoir (40), de telle sorte qu'un volume du réservoir (40) soit réduit lors de l'avance du piston (44), dans lequel le piston (44) est de préférence à ressort de manière à pousser le piston (44) dans une position normalement rétractée.

14. Système selon l'une quelconque des revendications 1 à 13, dans lequel ladite au moins une partie consommant de l'énergie de la section d'intestin artificiel (2) comprend au moins un moteur agencé pour entraîner automatiquement au moins une partie consommant de l'énergie supplémentaire de la section d'intestin artificiel (2).

15. Système selon la revendication 14, comprenant un commutateur pouvant être actionné manuellement destiné à activer le au moins un moteur, le commutateur étant agencé pour une implantation sous-cutanée de manière à pouvoir être actionné à partir de l'extérieur du corps (100) du patient.

16. Système selon l'une quelconque des revendications 1 à 15, comprenant un dispositif transformant l'énergie implantable destiné à transformer de manière sans fil une énergie transmise à partir de l'extérieur du corps (100) du patient en énergie devant être stockée dans l'accumulateur (A), et comprenant en outre un transmetteur d'énergie sans fil apte à transmettre de manière sans fil une énergie de l'extérieur du corps (100) du patient vers ledit dispositif de transformation d'énergie implantable.

17. Système selon la revendication 16, comprenant un sous-système de réaction apte à envoyer de manière sans fil des informations de réaction liées à l'énergie devant être stockée dans l'accumulateur (A) de l'intérieur du corps humain (100) à l'extérieur de celui-ci, dans lequel le système est apte à utiliser les informations de réaction pour adapter une quantité d'énergie sans fil transmise par le transmetteur d'énergie.

18. Système selon la revendication 17, dans lequel les informations de réaction sont liées à un bilan énergétique défini comme le bilan entre une quantité d'énergie sans fil reçue à l'intérieur du corps humain (100) et une quantité d'énergie consommée par la au moins une partie consommant de l'énergie, et/ou dans lequel les informations de réaction sont liées à un bilan énergétique défini comme le bilan entre un taux d'énergie sans fil reçue à l'intérieur du corps humain (100) et un taux d'énergie consommée par la au moins une partie consommant de l'énergie.

19. Système selon l'une quelconque des revendications 1 à 18, comprenant en outre une unité de commande (90, 91) apte à commander directement ou indirectement un ou plusieurs éléments du système, dans lequel l'unité de commande (90, 91) comprend une première partie (91) apte à l'implantation dans le corps (100) du patient et une seconde partie (90) apte à coopérer avec la première partie (91) à partir de l'extérieur du corps (100) du patient.

20. Système selon l'une quelconque des revendications 1 à 19, comprenant en outre un capteur de paramètre physique (93) apte à détecter directement ou indirectement un paramètre physique du patient, dans lequel le capteur de paramètre physique (93) est de préférence apte à détecter au moins un des paramètres physiques suivants du patient : une pression à l'intérieur de la section d'intestin artificiel (2) du patient, une pression à l'intérieur de l'intestin naturel (70, 80) du patient, une dilatation de la section d'intestin artificiel (2), une distension de la paroi intestinale de l'intestin naturel (70, 80) du patient, un mouvement de la paroi intestinale du patient, et/ou comprenant un capteur de paramètre fonctionnel apte à détecter directement ou indirectement un paramètre fonctionnel du système, dans lequel le capteur de paramètre fonctionnel est de préférence apte à détecter au moins un des paramètres fonctionnels suivants du système : une pression contre une partie du système comme la section d'intestin artificiel (2), une distension d'une partie du système comme une paroi de la section d'intestin artificiel (2), un paramètre électrique comme la tension, le courant ou le bilan énergétique, une position ou un mouvement d'une partie pouvant être déplacée du système.
